# EUROPEAN PATENT SPECIFICATION

(11) **EP 1 333 284 B1**
(45) Date of publication and mention of the grant of the patent: **16.11.2005**
(21) Application number: 03001652.1
(22) Date of filing: 24.01.2003
(51) Int. Cl.: G01N 33/543, C12Q 1/68, B01J 19/00

(54) **Method for conducting receptor-ligand association reactions**
Verfahren zur Durchführung von Rezeptor-Ligand Bindungsreaktionen
Procédé pour effectuer des réactions de liason entre recepteurs et ligands

(30) Priority: 04.02.2002 JP 2002026816
(43) Date of publication of application: 06.08.2003
(73) Proprietor: Fuji Photo Film Co. Ltd., Kanagawa 250-0193 (JP)
(72) Inventor: Nakajima, Kenji, Ashigarakami-gun, Kanagawa-ken (JP)
(74) Representative: Klunker . Schmitt-Nilson . Hirsch

(56) References cited:
- WO-A-00/45950
- WO-A-98/29736
- WO-A-02/094846
- WO-A-03/005013
- US-A1- 2001 026 917
- DATABASE WPI Derwent Publications Ltd., London, GB; AN 1999-378994 XP002242852 & JP 11 146784 A (DAINIPPON PRINTING CO LTD), 2 June 1999 (1999-06-02)

## Description

### BACKGROUND OF THE INVENTION

The present invention relates to a method for conducting a receptor-ligand association reaction and, particularly, to a method for conducting a receptor-ligand association reaction which can efficiently associate a receptor or ligand with ligands or receptors fixed in a biochemical analysis unit and produce biochemical analysis data having an excellent quantitative characteristic with good repeatability.

### DESCRIPTION OF THE PRIOR ART

An autoradiographic analyzing system using as a detecting material for detecting radiation a stimulable phosphor which can absorb, store and record the energy of radiation when it is irradiated with radiation and which, when it is then stimulated by an electromagnetic wave having a specified wavelength, can release stimulated emission whose light amount corresponds to the amount of radiation with which it was irradiated is known, which comprises the steps of introducing a radioactively labeled substance into an organism, using the organism or a part of the tissue of the organism as a specimen, superposing the specimen and a stimulable phosphor sheet formed with a stimulable phosphor layer for a certain period of time, storing and recording radiation energy in a stimulable phosphor contained in the stimulable phosphor layer, scanning the stimulable phosphor layer with an electromagnetic wave to excite the stimulable phosphor, photoelectrically detecting the stimulated emission released from the stimulable phosphor to produce digital image signals, effecting image processing on the obtained digital image signals, and reproducing an image on displaying means such as a CRT or the like or a photographic film (see, for example, Japanese Patent Publication No. 1-60784, Japanese Patent Publication No. 1-60782, Japanese Patent Publication No. 4-3952 and the like).

Unlike the autoradiographic analyzing system using a photographic film, according to the autoradiographic analyzing system using the stimulable phosphor as a detecting material, development, which is chemical processing, becomes unnecessary. Further, it is possible reproduce a desired image by effecting image processing on the obtained image data and effect quantitative analysis using a computer. Use of a stimulable phosphor in these processes is therefore advantageous.

On the other hand, a fluorescence analyzing system using a fluorescent substance as a labeling substance instead of a radioactive labeling substance in the autoradiographic analyzing system is known. According to this system, it is possible to study a genetic sequence, study the expression level of a gene, and to effect separation or identification of protein or estimation of the molecular weight or properties of protein or the like. For example, this system can perform a process including the steps of distributing a plurality of DNA fragments on a gel support by means of electrophoresis after a fluorescent dye was added to a solution containing a plurality of DNA fragments to be distributed, or distributing a plurality of DNA fragments on a gel support containing a fluorescent dye, or dipping a gel support on which a plurality of DNA fragments have been distributed by means of electrophoresis in a solution containing a fluorescent dye, thereby labeling the electrophoresed DNA fragments, exciting the fluorescent dye by a stimulating ray to cause it to release fluorescent light, detecting the released fluorescent light to produce an image and detecting the distribution of the DNA fragments on the gel support. This system can also perform a process including the steps of distributing a plurality of DNA fragments on a gel support by means of electrophoresis, denaturing the DNA fragments, transferring at least a part of the denatured DNA fragments onto a transfer support such as a nitrocellulose support by the Southern-blotting method, hybridizing a probe prepared by labeling target DNA and DNA or RNA complementary thereto with the denatured DNA fragments, thereby selectively labeling only the DNA fragments complementary to the probe DNA or probe RNA, exciting the fluorescent dye by a stimulating ray to cause it to release fluorescent light, detecting the released fluorescent light to produce an image and detecting the distribution of the target DNA on the transfer support. This system can further perform a process including the steps of preparing a DNA probe complementary to DNA containing a target gene labeled by a labeling substance, hybridizing it with DNA on a transfer support, combining an enzyme with the complementary DNA labeled by a labeling substance, causing the enzyme to contact a fluorescent substance, transforming the fluorescent substance to a fluorescent substance having fluorescent light releasing property, exciting the thus produced fluorescent substance by a stimulating ray to release fluorescent light, detecting the fluorescent light to produce an image and detecting the distribution of the target DNA on the transfer support. This fluorescence detecting system is advantageous in that a genetic sequence or the like can be easily detected without using a radioactive substance.

Similarly, there is known a chemiluminescence analyzing system comprising the steps of fixing specific binding substances such as a protein, a nucleic acid or the like in a biochemical analysis unit such as a membrane filter, specifically binding a substance derived from a living organism and labeled with a labeling substance which generates chemiluminescent emission when it contacts a chemiluminescent substrate with specific binding substances, thereby selectively labeling the specific binding substances, bringing the specific binding substances and the substance derived from a living organism and specifically bound with the specific binding substances into contact with the chemiluminescent substrate, photoelectrically detecting the chemiluminescent emission in the wavelength of visible light generated by the contact of the chemiluminescent substrate and the labeling substance to produce digital image signals, effecting image processing thereon, and reproducing a chemiluminescent image on a display means such as a CRT or a recording material such as a photographic film, thereby obtaining information relating to the high molecular substance such as genetic information.

Further, a micro-array analyzing system has been recently developed, which comprises the steps of using a spotting device to drop at different positions on the surface of a carrier such as a slide glass plate, a membrane filter or the like specific binding substances, which can specifically bind with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substances using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a labeling substance such as a fluorescent substance, dye or the like, thereby forming a micro-array, irradiating the micro-array with a stimulating ray, photoelectrically detecting light such as fluorescence emission released from a labeling substance such as a fluorescent substance, dye or the like, and analyzing the substance derived from a living organism. This micro-array analyzing system is advantageous in that a substance derived from a living organism can be analyzed in a short time period by forming a number of spots of specific binding substances at different positions of the surface of a carrier such as a slide glass plate at high density and hybridizing them with a substance derived from a living organism and labeled with a labeling substance.

In addition, a macro-array analyzing system using a radioactive labeling substance as a labeling substance has been further developed, which comprises the steps of using a spotting device to drop at different positions on the surface of a carrier such as a membrane filter or the like specific binding substances, which can specifically bind with a substance derived from a living organism such as a cell, virus,- hormone, tumor marker, enzyme, antibody, antigen, abzyme, other protein, a nuclear acid, cDNA, DNA, RNA or the like and whose sequence, base length, composition and the like are known, thereby forming a number of independent spots, specifically binding the specific binding substance using a hybridization method or the like with a substance derived from a living organism such as a cell, virus, hormone, tumor marker, enzyme, antibody, antigen, abzyme, other protein, a nuclear acid, cDNA, DNA or mRNA by extraction, isolation or the like and optionally further subjected to chemical processing, chemical modification or the like and which is labeled with a radioactive labeling substance, thereby forming a macro-array, superposing the macro-array and a stimulable phosphor sheet formed with a stimulable phosphor layer, exposing the stimulable phosphor layer to a radioactive labeling substance, irradiating the stimulable phosphor layer with a stimulating ray to excite the stimulable phosphor, photoelectrically detecting the stimulated emission released from the stimulable phosphor to produce biochemical analysis data, and analyzing the substance derived from a living organism.

In the micro-array analyzing system and the macro-array analyzing system, it is required to produce biochemical analysis data by dropping a solution containing specific binding substances at different positions on the surface of a biochemical analysis unit such as a membrane filter or the like to form a number of spot-like regions, hybridizing a substance derived from a living organism and labeled with a labeling substance such as a radioactive labeling substance, a fluorescent substance or a labeling substance which generates chemiluminescence emission when it contacts a chemiluminescent substrate with the specific binding substances contained in the spot-like regions, thereby selectively labeling the spot-like regions, exposing a stimulable phosphor layer of a stimulable phosphor sheet to a radioactive labeling substance selectively contained in the spot-like regions, scanning the thus exposed stimulable phosphor layer with a stimulating ray, thereby exciting stimulable phosphor contained in the stimulable phosphor layer and photoelectrically detecting stimulated emission released from the stimulable phosphor, or scanning a number of the spot-like regions with a stimulating ray to produce biochemical analysis data, thereby exciting a fluorescent substance contained in a number of the spot-like regions and photoelectrically detecting fluorescence emission released from the fluorescent substance to produce biochemical analysis data, or bringing a labeling substance contained in a number of the spot-like regions into contact with a chemiluminescent substrate and photoelectrically detecting chemiluminescence emission released from the labeling substance to produce biochemical analysis data.

Conventionally, hybridization of specific binding substances and a substance derived from a living organism has been performed by an experimenter manually inserting a biochemical analysis unit formed with a number of the spot-like regions containing specific binding substances such as a membrane filter into a hybridization bag, pouring a hybridization reaction solution containing a substance derived from a living organism and labeled with a labeling substance such as a radioactive labeling substance, a fluorescent substance or a labeling substance which generates chemiluminescence emission when it contacts a chemiluminescent substrate into the hybridization bag, vibrating the hybridization bag, thereby moving the substance derived from a living organism by convection or diffusion, hybridizing the substance derived from a living organism with the specific binding substances, removing the biochemical analysis unit from the hybridization bag, and inserting the biochemical analysis unit in a container filled with a cleaning solution, thereby cleaning the biochemical analysis unit.

However, in the case where specific binding substances and a substance derived from a living organism are hybridized by an experimenter manually inserting a biochemical analysis unit into a hybridization bag, pouring a hybridization reaction solution into the hybridization bag, and vibrating the hybridization bag, it is difficult to bring the hybridization reaction solution into uniform contact with a number of the spot-like regions containing specific binding substances and, therefore, specific binding substances and a substance derived from a living organism cannot be effectively hybridized.

Further, in the case of an experimenter manually inserting a biochemical analysis unit into a hybridization bag by, pouring a hybridization reaction solution into the hybridization bag, vibrating the hybridization bag, hybridizing specific binding substances and a substance derived from a living organism, removing the biochemical analysis unit from the hybridization bag, and inserting the biochemical analysis unit in a container filled with a cleaning solution, thereby cleaning the biochemical analysis unit, the results of the hybridization differ among different experimenters and the repeatability of the hybridization is inevitably lowered. Moreover, even when the same experimenter performs hybridization, different results may be obtained.

The same problems occur in the case where a receptor and a ligand are associated as in the case of fixing antigens or antibodies in a biochemical analysis unit such as a membrane filter and binding an antibody or an antigen to the thus fixed antigens or antibodies by an antigen-antibody reaction and the same problems occur in the case of hybridizing a probe DNA labeled with a hapten such as digoxigenin with a target DNA fixed in a biochemical analysis unit such as a membrane filter, binding an antibody for the hapten such as digoxigenin labeled with an enzyme which generates chemiluminescent emission when it contacts a chemiluminescent substrate or an antibody for the hapten such as digoxigenin labeled with an enzyme which generates fluorescence emission when it contacts a fluorescent substrate with the hapten labeling the probe DNA by an antigen-antibody reaction, thereby labeling the target DNA.

WO-A-02/094846 and WO-A-03/005013 are state of the art according to Art. 54 (3) EPC. Both documents disclose a method for carrying out high-throughput binding assays using a flow-through microarray chip, in which capture molecules are arrayed on absorptive spots and a solution containing a labeled target molecule is forced to pass through the absorptive spots (whereby the capture molecule binds to the capture molecule) by means of an applied pressure differential across both sides of the chip. In the device of WO-A-02/094846, the absorptive spots are formed by placing a porous sheet on the top of a substrate having corresponding through-holes. In the device of WO-A-03/005013, the absorptive spots containing the capture reagents are formed by printing the reagents on the surface of a porous nitrocellulose membrane and this printed membrane is sandwiched between the two members of a holder. WO-A-00/45950 discloses a method for carrying out polymer synthesis using a flow-through analysis unit comprising a functionalized absorptive membrane sandwiched between two substrates having corresponding through-holes.

### SUMMARY OF THE INVENTION

It is therefore an object of the present invention to provide a method for conducting a receptor-ligand association reaction which can efficiently associate a ligand or receptor with receptors or ligands fixed in spot-like regions of a biochemical analysis unit and produce biochemical analysis data having an excellent quantitative characteristic with good repeatability. This is accomplished by a method having the features of claim 1.

The above other objects of the present invention can be accomplished by a method for conducting a receptor-ligand association reaction comprising the steps of pressurizing a reaction solution containing a ligand or receptor labeled with a labeling substance and forcibly feeding the reaction solution so as to cut through a plurality of absorptive regions formed in a biochemical analysis unit so as to be spaced from each other and containing receptors or ligands, thereby selectively associating the ligand or receptor contained in the reaction solution with the receptors or ligands contained in the plurality of absorptive regions of the biochemical analysis unit.

In the present invention, the receptor-ligand association reaction includes a hybridization reaction and an antigen-antibody reaction.

According to the present invention, since the method for conducting a receptor-ligand association reaction includes the steps of pressurizing a reaction solution containing a ligand or receptor labeled with a labeling substance and forcibly feeding the reaction solution so as to cut through a plurality of absorptive regions formed in a biochemical analysis unit so as to be spaced from each other and containing receptors or ligands, thereby selectively associating the ligand or receptor contained in the reaction solution with the receptors or ligands contained in the plurality of absorptive regions of the biochemical analysis unit, it is possible to markedly increase the moving rate of the ligand or receptor through the plurality of absorptive regions of the biochemical analysis unit. Therefore, since it is possible to markedly increase the reaction rate of association of the receptors or ligands contained in the plurality of absorptive regions of the biochemical analysis unit and the ligand or receptor contained in the reaction solution and it is further possible to markedly increase the possibility of association of the ligand or receptor contained in the reaction solution with the receptors or ligands fixed in deep portions of the plurality of absorptive regions of the biochemical analysis unit, the ligand or receptor contained in the reaction solution can be associated with the receptors or ligands contained in the plurality of absorptive regions of the biochemical analysis unit in a desired manner.

In a preferred aspect of the present invention, the method for conducting a receptor-ligand association reaction includes the step of pressurizing the reaction solution using a syringe, thereby forcibly feeding the reaction solution so as to cut through the plurality of absorptive regions formed in the biochemical analysis unit.

According to this preferred aspect of the present invention, since the method for conducting a receptor-ligand association reaction includes the step of pressurizing the reaction solution using a syringe, thereby forcibly feeding the reaction solution so as to cut through the plurality of absorptive regions formed in the biochemical analysis unit, it is possible to markedly increase the moving rate of the ligand or receptor through the plurality of absorptive regions of the biochemical analysis unit. Therefore, since it is possible to markedly increase the reaction rate of association of the receptors or ligands contained in the plurality of absorptive regions of the biochemical analysis unit and the ligand or receptor contained in the reaction solution and it is further possible to markedly increase the possibility of association of the ligand or receptor contained in the reaction solution with the receptors or ligands fixed in deep portions of the plurality of absorptive regions of the biochemical analysis unit, the ligand or receptor contained in the reaction solution can be associated with the receptors or ligands contained in the plurality of absorptive regions of the biochemical analysis unit in a desired manner.

In another preferred aspect of the present invention, the method for conducting a receptor-ligand association reaction includes the step of deforming diaphragms facing each other to pressurize the reaction solution, thereby forcibly feeding the reaction solution so as to cut through the plurality of absorptive regions formed in the biochemical analysis unit.

According to this preferred aspect of the present invention, since the method for conducting a receptor-ligand association reaction includes the step of deforming diaphragms facing each other to pressurize the reaction solution, thereby forcibly feeding the reaction solution so as to cut through the plurality of absorptive regions formed in the biochemical analysis unit, it is possible to markedly increase the moving rate of the ligand or receptor through the plurality of absorptive regions of the biochemical analysis unit. Therefore, since it is possible to markedly increase the reaction rate of association of the receptors or ligands contained in the plurality of absorptive regions of the biochemical analysis unit and the ligand or receptor contained in the reaction solution and it is further possible to markedly increase the possibility of association of the ligand or receptor contained in the reaction solution with the receptors or ligands fixed in deep portions of the plurality of absorptive regions of the biochemical analysis unit, the ligand or receptor contained in the reaction solution can be associated with the receptors or ligands contained in the plurality of absorptive regions of the biochemical analysis unit in a desired manner.

In a preferred aspect of the present invention, the reaction solution contains a ligand or receptor labeled with at least one kind of labeling substance selected from a group consisting of a radioactive labeling substance, a fluorescent substance and a labeling substance which generates chemiluminescence emission when it contacts a chemiluminescent substrate.

In a preferred aspect of the present invention, the method for conducting a receptor-ligand association reaction includes the steps of pressurizing a reaction solution containing a substance derived from a living organism and labeled with a labeling substance and forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with a labeling substance with the specific binding substances contained in the plurality of absorptive regions of the biochemical analysis unit.

In another preferred aspect of the present invention, the method for conducting a receptor-ligand association reaction includes the steps of pressurizing a reaction solution containing an antibody or an antigen labeled with a labeling substance and forcibly feeding the reaction solution so as to cut through the plurality of absorptive regions formed in the biochemical analysis unit so as to be spaced apart from each other and containing an antigen or an antibody, thereby binding the antibody or the antigen labeled with a labeling substance with the antigen or the antibody contained in the plurality of absorptive regions of the biochemical analysis unit.

In another preferred aspect of the present invention, the method for conducting a receptor-ligand association reaction includes the steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, pressurizing an antibody solution containing an antibody to the hapten labeled with a labeling substance and forcibly feeding the antibody solution so as to cut through the plurality of absorptive regions of the biochemical analysis unit, thereby binding the antibody labeled with the labeling substance with the hapten contained in the plurality of absorptive regions of the biochemical analysis unit by an antigen-antibody reaction.

In the present invention, illustrative examples of the combination of hapten and antibody include digoxigenin and anti-digoxigenin antibody, theophylline and anti-theophylline antibody, fluorosein and anti-fluorosein antibody, and the like. Further, the combination of biotin and avidin, antigen and antibody may be utilized instead of the combination of hapten and antibody.

In a further preferred aspect of the present invention, the method for conducting a receptor-ligand association reaction includes the steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, and pressurizing an antibody solution containing an antibody to the hapten labeled with an enzyme which generates chemiluminescence emission when it contacts a chemiluminescent substrate so as to cut through the plurality of absorptive regions formed in the biochemical analysis unit, thereby binding the antibody labeled with the enzyme with the hapten contained in the plurality of absorptive regions of the biochemical analysis unit by an antigen-antibody reaction.

In another preferred aspect of the present invention, the method for conducting a receptor-ligand association reaction includes the steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, and pressurizing an antibody solution containing an antibody to the hapten labeled with an enzyme which generates a fluorescent substance when it contacts a fluorescent substrate so as to cut through the plurality of absorptive regions formed in the biochemical analysis unit, thereby binding the antibody labeled with the enzyme with the hapten contained in the plurality of absorptive regions of the biochemical analysis unit by an antigen-antibody reaction.

In a preferred aspect of the present invention, the biochemical analysis unit includes a substrate formed with a plurality of through-holes spaced apart from each other and the plurality of absorptive regions are formed by charging an absorptive material in the plurality of through-holes formed in the substrate and causing the absorptive material charged in the plurality of through-holes to contain receptors or ligands.

In a further preferred aspect of the present invention, the plurality of absorptive regions are formed by embedding an absorptive material in the plurality of through-holes formed in the substrate and causing the absorptive material embedded in the plurality of through-holes to contain receptors or ligands.

In another preferred aspect of the present invention, the plurality of absorptive regions are formed by pressing an absorptive membrane containing an absorptive material into the plurality of through-holes formed in the substrate and causing the absorptive material pressed into the plurality of through-holes to contain receptors or ligands.

In another preferred aspect of the present invention, the biochemical analysis unit includes an absorptive substrate formed of an absorptive material and a substrate formed with a plurality of through-holes and being in close contact with at least one surface of the absorptive substrate and the plurality of absorptive regions of the biochemical analysis unit are formed by causing the absorptive substrate within the plurality of through-holes formed in the substrate to contain receptors or ligands.

In a preferred aspect of the present invention, the biochemical analysis unit is formed with 10 or more absorptive regions.

In a further preferred aspect of the present invention, the biochemical analysis unit is formed with 50 or more absorptive regions.

In a further preferred aspect of the present invention, the biochemical analysis unit is formed with 100 or more absorptive regions.

In a further preferred aspect of the present invention, the biochemical analysis unit is formed with 500 or more absorptive regions.

In a further preferred aspect of the present invention, the biochemical analysis unit is formed with 1,000 or more absorptive regions.

In a further preferred aspect of the present invention, the biochemical analysis unit is formed with 5,000 or more absorptive regions.

In a further preferred aspect of the present invention, the biochemical analysis unit is formed with 10,000 or more absorptive regions.

In a further preferred aspect of the present invention, the biochemical analysis unit is formed with 50,000 or more absorptive regions.

In a further preferred aspect of the present invention, the biochemical analysis unit is formed with 100,000 or more absorptive regions.

In a preferred aspect of the present invention, each of the plurality of absorptive regions of the biochemical analysis unit has a size of less than 5 mm².

In a further preferred aspect of the present invention, each of the plurality of absorptive regions of the biochemical analysis unit has a size of less than 1 mm².

In a further preferred aspect of the present invention, each of the plurality of absorptive regions of the biochemical analysis unit has a size of less than 0.5 mm².

In a further preferred aspect of the present invention, each of the plurality of absorptive regions of the biochemical analysis unit has a size of less than 0.1 mm².

In a further preferred aspect of the present invention, each of the plurality of absorptive regions of the biochemical analysis unit has a size of less than 0.05 mm².

In a further preferred aspect of the present invention, each of the plurality of absorptive regions of the biochemical analysis unit has a size of less than 0.01 mm².

In a preferred aspect of the present invention, the plurality of absorptive regions are formed in the biochemical analysis unit at a density of 10 or more per cm².

In a further preferred aspect of the present invention, the plurality of absorptive regions are formed in the biochemical analysis unit at a density of 50 or more per cm².

In a further preferred aspect of the present invention, the plurality of absorptive regions are formed in the biochemical analysis unit at a density of 100 or more per cm².

In a further preferred aspect of the present invention, the plurality of absorptive regions are formed in the biochemical analysis unit at a density of 500 or more per cm².

In a further preferred aspect of the present invention, the plurality of absorptive regions are formed in the biochemical analysis unit at a density of 1,000 or more per cm².

In a further preferred aspect of the present invention, the plurality of absorptive regions are formed in the biochemical analysis unit at a density of 5,000 or more per cm².

In a further preferred aspect of the present invention, the plurality of absorptive regions are formed in the biochemical analysis unit at a density of 10,000 or more per cm².

In a further preferred aspect of the present invention, the plurality of absorptive regions are formed in the biochemical analysis unit at a density of 50,000 or more per cm².

In a further preferred aspect of the present invention, the plurality of absorptive regions are formed in the biochemical analysis unit at a density of 100,000 or more per cm².

In a preferred aspect of the present invention, the plurality of absorptive regions are formed in the biochemical analysis unit in a regular pattern.

In a preferred aspect of the present invention, each of the plurality of through-holes is formed substantially circular in the substrate of the biochemical analysis unit.

In the present invention, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, the substrate of the biochemical analysis unit preferably has a property of attenuating radiation energy.

In this preferred aspect of the present invention, since the substrate of the biochemical analysis unit has a property of attenuating radiation energy, even in the case of forming the plurality of absorptive regions in the biochemical analysis unit at a high density, selectively hybridizing a substance derived from a living organism and labeled with a radioactive labeling substance with specific binding substances contained in the plurality of absorptive regions, thereby selectively labeling the plurality of absorptive regions with the radioactive labeling substance, and superposing the biochemical analysis unit and a stimulable phosphor sheet formed with a stimulable phosphor layer to expose the stimulable phosphor layer formed in the stimulable phosphor sheet to the radioactive labeling substance selectively contained in the plurality of absorptive regions of the biochemical analysis unit, thereby recording radiation data in the stimulable phosphor layer of the stimulable phosphor sheet, electron beams (β rays) released from the radioactive labeling substance contained in the individual absorptive regions of the biochemical analysis unit can be effectively prevented from scattering in the substrate of the biochemical analysis unit. Therefore, since it is possible to cause electron beams (β rays) to selectively enter a corresponding region of the stimulable phosphor layer to expose only the corresponding regions of the stimulable phosphor layer thereto, it is possible to produce biochemical analysis data having an excellent quantitative characteristic with high resolution by scanning the plurality of thus exposed stimulable phosphor layer regions with a stimulating ray and photoelectrically detecting stimulated emission released from the plurality of stimulable phosphor layer regions.

In a preferred aspect of the present invention, the substrate of the biochemical analysis unit has a property of reducing the energy of radiation to 1/5 or less when the radiation travels in the substrate by a distance equal to that between neighboring absorptive regions.

In a further preferred aspect of the present invention, the substrate of the biochemical analysis unit has a property of reducing the energy of radiation to 1/10 or less when the radiation travels in the substrate by a distance equal to that between neighboring absorptive regions.

In a further preferred aspect of the present invention, the substrate of the biochemical analysis unit has a property of reducing the energy of radiation to 1/50 or less when the radiation travels in the substrate by a distance equal to that between neighboring absorptive regions.

In a further preferred aspect of the present invention, the substrate of the biochemical analysis unit has a property of reducing the energy of radiation to 1/100 or less when the radiation travels in the substrate by a distance equal to that between neighboring absorptive regions.

In a further preferred aspect of the present invention, the substrate of the biochemical analysis unit has a property of reducing the energy of radiation to 1/500 or less when the radiation travels in the substrate by a distance equal to that between neighboring absorptive regions.

In a further preferred aspect of the present invention, the substrate of the biochemical analysis unit has a property of reducing the energy of radiation to 1/1,000 or less when the radiation travels in the substrate by a distance equal to that between neighboring absorptive regions.

In the present invention, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, the substrate of the biochemical analysis unit preferably has a property of attenuating light energy.

In this preferred aspect of the present invention, since the substrate of the biochemical analysis unit has a property of attenuating light energy, even in the case of forming the plurality of absorptive regions in the biochemical analysis unit at a high density, selectively binding a substance derived from a living organism and labeled with a fluorescent substance or a labeling substance which generates chemiluminescence emission when it contacts a chemiluminescent substrate, irradiating the plurality of absorptive regions with a stimulating ray or bringing the plurality of absorptive regions into contact with a chemiluminescent substrate, and photoelectrically detecting fluorescence emission or chemiluminescence emission released from the plurality of absorptive regions to produce biochemical analysis data, fluorescence emission or chemiluminescence emission released from a particular absorptive region of the biochemical analysis unit can be effectively prevented from scattering in the substrate of the biochemical analysis unit and mixing fluorescence emission or chemiluminescence emission released from neighboring absorptive regions. Therefore, it is possible to produce biochemical analysis data having an excellent quantitative characteristic by photoelectrically detecting fluorescence emission or chemiluminescence emission released from the plurality of absorptive regions of the biochemical analysis unit.

In a preferred aspect of the present invention, the substrate of the biochemical analysis unit has a property of reducing the energy of light to 1/5 or less when the light travels in the substrate by a distance equal to that between neighboring absorptive regions.

In a further preferred aspect of the present invention, the substrate of the biochemical analysis unit has a property of reducing the energy of light to 1/10 or less when the light travels in the substrate by a distance equal to that between neighboring absorptive regions.

In a further preferred aspect of the present invention, the substrate of the biochemical analysis unit has a property of reducing the energy of light to 1/50 or less when the light travels in the substrate by a distance equal to that between neighboring absorptive regions.

In a further preferred aspect of the present invention, the substrate of the biochemical analysis unit has a property of reducing the energy of light to 1/100 or less when the light travels in the substrate by a distance equal to that between neighboring absorptive regions.

In a further preferred aspect of the present invention, the substrate of the biochemical analysis unit has a property of reducing the energy of light to 1/500 or less when the light travels in the substrate by a distance equal to that between neighboring absorptive regions.

In a further preferred aspect of the present invention, the substrate of the biochemical analysis unit has a property of reducing the energy of light to 1/1,000 or less when the light travels in the substrate by a distance equal to that between neighboring absorptive regions.

In the present invention, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, a material for forming the substrate of the biochemical analysis unit preferably has a property of attenuating radiation energy and/or light energy but is not particularly limited. The material for forming the substrate of the biochemical analysis unit may be any type of inorganic compound material or organic compound material and the substrate of the biochemical analysis unit can preferably be formed of a metal material, a ceramic material or a plastic material.

In the present invention, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, illustrative examples of inorganic compound materials preferably usable for forming the substrate of the biochemical analysis unit in the present invention include metals such as gold, silver, copper, zinc, aluminum, titanium, tantalum, chromium, iron, nickel, cobalt, lead, tin, selenium and the like; alloys such as brass, stainless steel, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like.

In the present invention, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, a high molecular compound can preferably be used as an organic compound material preferably usable for forming the substrate of the biochemical analysis unit. Illustrative examples of high molecular compounds preferably usable for forming the substrate of the biochemical analysis unit in the present invention include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4,10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Since the capability of attenuating radiation energy generally increases as specific gravity increases, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, the substrate of the biochemical analysis unit is preferably formed of a compound material or a composite material having specific gravity of 1.0 g/cm³ or more and more preferably formed of a compound material or a composite material having specific gravity of 1.5 g/cm³ to 23 g/cm³.

Further, since the capability of attenuating light energy generally increases as scattering and/or absorption of light increases, in the case where the plurality of absorptive regions of the biochemical analysis unit are formed by charging an absorptive material in the plurality of through-holes formed in the substrate and causing the absorptive material charged in the plurality of through-holes to contain receptors or ligands or where the plurality of absorptive regions of the biochemical analysis unit are formed by causing an absorptive substrate within the plurality of through-holes formed in the substrate to contain receptors or ligands, the substrate of the biochemical analysis unit preferably has absorbance of 0.3 per cm (thickness) or more and more preferably has absorbance of 1 per cm (thickness) or more. The absorbance can be determined by placing an integrating sphere immediately behind a platelike member having a thickness of T cm, measuring an amount A of transmitted light at a wavelength of probe light or emission light used for measurement by a spectrophotometer, and calculating A/T. In the present invention, a light scattering substance or a light absorbing substance may be added to the substrate of the biochemical analysis unit in order to improve the capability of attenuating light energy. Particles of a material different from a material forming the substrate of the biochemical analysis unit may be preferably used as a light scattering substance and a pigment or dye may be preferably used as a light absorbing substance.

In another preferred aspect of the present invention, the biochemical analysis unit includes an absorptive substrate formed of an absorptive material and the plurality of absorptive regions are formed by causing different positions of the absorptive substrate to contain receptors or ligands.

In the present invention, a porous material or a fiber material may be preferably used as the absorptive material for forming the absorptive regions or the absorptive substrate of the biochemical analysis unit. The absorptive regions or the absorptive substrate may be formed by combining a porous material and a fiber material.

In the present invention, a porous material for forming the absorptive regions or the absorptive substrate of the biochemical analysis unit may be any type of an organic material or an inorganic material and may be an organic/inorganic composite material.

In the present invention, an organic porous material used for forming the absorptive regions or the absorptive substrate of the biochemical analysis unit is not particularly limited but a carbon porous material such as an activated carbon or a porous material capable of forming a membrane filter is preferably used. Illustrative examples of porous materials capable of forming a membrane filter include nylons such as nylon-6, nylon-6,6, nylon-4,10; cellulose derivatives such as nitrocellulose, acetyl cellulose, butyric-acetyl cellulose; collagen; alginic acids such as alginic acid, calcium alginate, alginic acid/poly-L-lysine polyionic complex; polyolefins such as polyethylene, polypropylene; polyvinyl chloride; polyvinylidene chloride; polyfluoride such as polyvinylidene fluoride, polytetrafluoride; and copolymers or composite materials thereof.

In the present invention, an inorganic porous material used for forming the absorptive regions or the absorptive substrate of the biochemical analysis unit is not particularly limited. Illustrative examples of inorganic porous materials preferably usable in the present invention include metals such as platinum, gold, iron, silver, nickel, aluminum and the like; metal oxides such as alumina, silica, titania, zeolite and the like; metal salts such as hydroxy apatite, calcium sulfate and the like; and composite materials thereof.

In the present invention, a fiber material used for forming the absorptive regions or the absorptive substrate of the biochemical analysis unit is not particularly limited. Illustrative examples of fiber materials preferably usable in the present invention include nylons such as nylon-6, nylon-6,6, nylon-4,10; and cellulose derivatives such as nitrocellulose, acetyl cellulose, butyric-acetyl cellulose.

In the present invention, the absorptive regions of the biochemical analysis unit may be formed using an oxidization process such as an electrolytic process, a plasma process, an arc discharge process or the like; a primer process using a silane coupling agent, titanium coupling agent or the like; and a surface-active agent process or the like.

The above and other objects and features of the present invention will become apparent from the following description made with reference to the accompanying drawings.

### BRIEF DESCRIPTION OF THE DRAWINGS

Figure 1 is a schematic perspective view showing a biochemical analysis unit used in a method for conducting a receptor-ligand association reaction which is a preferred embodiment of the present invention.
Figure 2 is a schematic front view showing a spotting device.
Figure 3 is a schematic longitudinal cross sectional view showing a reactor used for conducting a receptor-ligand association reaction which is a preferred embodiment of the present invention.
Figure 4 is a schematic perspective view showing a stimulable phosphor sheet.
Figure 5 is a schematic cross-sectional view showing a method for exposing a number of stimulable phosphor layer regions formed in a stimulable phosphor sheet to a radioactive labeling substance contained in a number of absorptive regions formed in a biochemical analysis unit.
Figure 6 is a schematic view showing a scanner for reading radiation data of a radioactive labeling substance recorded in a number of stimulable phosphor layer regions formed in a stimulable phosphor sheet and fluorescence data recorded in a number of absorptive regions formed in a biochemical analysis unit to produce biochemical analysis data.
Figure 7 is a schematic perspective view showing details in the vicinity of a photomultiplier of a scanner shown in Figure 6.
Figure 8 is a schematic cross-sectional view taken along a line A-A in Figure 7.
Figure 9 is a schematic cross-sectional view taken along a line B-B in Figure 7.
Figure 10 is a schematic cross-sectional view taken along a line C-C in Figure 7.
Figure 11 is a schematic cross-sectional view taken along a line D-D in Figure 7.
Figure 12 is a schematic plan view showing a scanning mechanism of an optical head.
Figure 13 is a block diagram of a control system, an input system, a drive system and a detection system of the scanner shown in Figure 7.
Figure 14 is a schematic front view showing a data producing system for reading chemiluminescence data recorded in a number of the absorptive regions formed in a biochemical analysis unit, and producing biochemical analysis data.
Figure 15 is a schematic longitudinal cross sectional view showing a cooled CCD camera of a data producing system.
Figure 16 is a schematic vertical cross sectional view showing a dark box of a data producing system.
Figure 17 is a block diagram of a personal computer of a data producing system and peripheral devices thereof.
Figure 18 is a schematic longitudinal cross-sectional view showing an reactor used for conducting a receptor-ligand association reaction, which is another preferred embodiment of the present invention.

### DESCRIPTION OF THE PREFERRED EMBODIMENTS

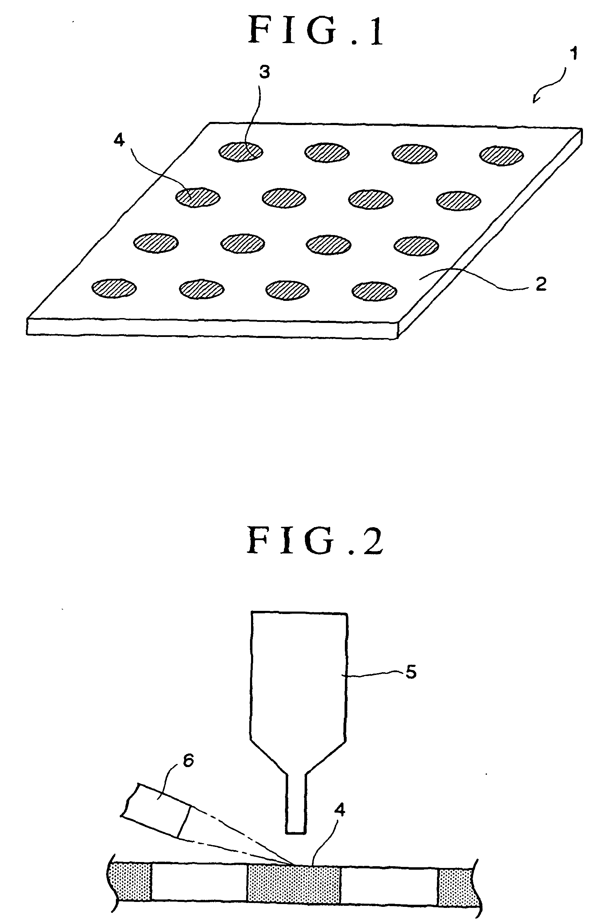

Figure 1 is a schematic perspective view showing a biochemical analysis unit used in a method for conducting a receptor-ligand association reaction which is a preferred embodiment of the present invention.

As shown in Figure 1, a biochemical analysis unit 1 includes a substrate 2 made of stainless steel and formed with a number of substantially circular through-holes 3 at a high density and a number of dot-like absorptive regions 4 are formed by charging nylon-6, 6 in a number of the through-holes 3.

Although not accurately shown in Figure 1, in this embodiment, the substantially circular through-holes 3 having a size of about 0.01 mm² are regularly formed in the substrate 2 in the manner of a matrix of 120 columns x 160 lines and, therefore, 19,200 absorptive regions 4 are formed. A number of absorptive regions 4 are formed by charging nylon-6, 6 in the through-holes 3 formed in the substrate in such a manner that the surfaces of the absorptive regions 4 are located at the same height level as that of the substrate.

When biochemical analysis is to be performed, a solution containing specific binding substances such as a plurality of cDNAs whose sequences are known but differ from each other are spotted using a spotting device onto a number of the absorptive regions 4 of the biochemical analysis unit 1 and the specific binding substances are absorbed therein.

Figure 2 is a schematic front view showing a spotting device.

As shown in Figure 2, the spotting device includes an injector 5 for ejecting a solution of specific binding substances toward the biochemical analysis unit 1 and a CCD camera 6 and is constituted so that the solution of specific binding substances such as cDNAs each of which has a known base sequence and is different from the others are spotted from the injector 6 when the tip end portion of the injector 5 and the center of the absorptive region 4 into which the solution containing specific binding substances is to be spotted are determined to coincide with each other as a result of viewing them using the CCD camera 6, thereby ensuring that the solution of specific binding substances can be accurately spotted into a number of the dot-like absorptive regions 4 of the biochemical analysis unit 1.

A substance derived from a living organism and labeled with a labeling substance is then hybridized with the specific binding substances such as cDNAs absorbed in a number of the absorptive regions 4 formed in the substrate 2 of the biochemical analysis unit 1.

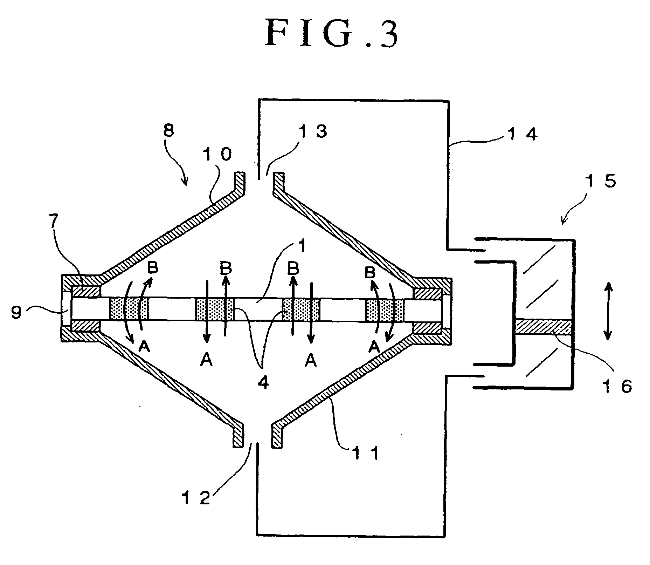

Figure 3 is a schematic longitudinal cross sectional view showing a reactor used for conducting a receptor-ligand association reaction which is a preferred embodiment of the present invention.

As shown in Figure 3, the reactor according to this embodiment includes a reaction vessel 8 in which a biochemical analysis unit holding section 7 is provided for holding the biochemical analysis unit 1. The biochemical analysis unit holding section 7 is constituted so as to be able to prevent leakage of a liquid.

The reaction vessel 8 includes a main body 9, an upper half portion 10 and a lower half portion 11. The upper half portion 10 is detachably attached to the main body 9 of the reaction vessel 8.

Therefore, the biochemical analysis unit 1 can be set in the reaction vessel 8 by removing the upper half portion 10 from the main body 9 of the reaction vessel 8.

As shown in Figure 3, a substantially center portion of the bottom wall of the lower half portion 8c of the reaction vessel 8 is formed with a solution feeding opening 12 through which a solution can be fed into the reaction vessel 8 and a substantially center portion of the top wall of the upper half portion 8b of the reaction vessel 8 is formed with a solution flow-in and flow-out opening 13 through which a solution can be fed into and discharged from the reaction vessel 8.

As shown in Figure 3, a solution circulation pipe 14 is detachably attached to the solution feeding opening 12 and the solution flow-in and flow-out opening 13. A syringe 16 including a piston 15 is provided in the solution circulation pipe 14.

In the thus constituted reactor according to this embodiment, a substance derived from a living body, labeled with a labeling substance and contained in a hybridization reaction solution selectively hybridizes specific binding substances contained in a number of the absorptive regions 4 of the biochemical analysis unit 1 in the following manner.

The upper half portion 10 is first removed from the main body 9 of the reaction vessel 8 and the biochemical analysis unit 1 formed with a number of the absorptive regions 4 in which specific binding substances are absorbed is set by a user at the biochemical analysis unit holding section 7 in the reaction vessel 8.

The upper half portion 10 is then attached to the main body 9 of the reaction vessel 8 and a hybridization reaction solution is prepared and fed into the reaction vessel 8.

In the case where a specific binding substance such as cDNA is to be labeled with a radioactive labeling substance, a hybridization reaction solution containing a substance derived from a living organism and labeled with a radioactive labeling substance as a probe is prepared.

Further, in the case where a specific binding substance such as cDNA is to be labeled with a fluorescent substance, a hybridization reaction solution containing a substance derived from a living organism and labeled with a fluorescent substance as a probe is prepared.

On the other hand, in the case where a specific binding substance such as cDNA is to be labeled with a labeling enzyme which generates chemiluminescence emission when it contacts a chemiluminescent substrate, a hybridization reaction solution 19 containing a substance derived from a living organism and labeled with a hapten such as digoxigenin as a probe is prepared.

It is possible to prepare a hybridization reaction solution containing two or more substances derived from a living organism among a substance derived from a living organism and labeled with a radioactive labeling substance, a substance derived from a living organism and labeled with a fluorescent substance such as a fluorescent dye and a substance derived from a living organism and labeled with a hapten such as digoxigenin. In this embodiment, a hybridization reaction solution containing a substance derived from a living organism and labeled with a radioactive labeling substance, a substance derived from a living organism and labeled with a fluorescent substance such as a fluorescent dye and a substance derived from a living organism and labeled with a hapten such as digoxigenin is prepared.

The thus prepared hybridization reaction solution is fed into the reaction vessel 8 through the solution feeding opening 12 formed in the lower half portion 11 of the reaction vessel 8 and when the inside of the reaction vessel 8 has been filled with the hybridization reaction solution, the solution circulation pipe 14 is attached to the solution feeding opening 12 formed in the lower half portion 11 of the reaction vessel 8.

A piston motor (not shown) is driven for driving the piston 15 of the syringe 16 so that the piston 15 is moved upward in Figure 3.

When the piston 15 of the syringe 16 is moved upward in Figure 3, the hybridization reaction solution contained in the space above the biochemical analysis unit 1 held at the biochemical analysis unit holding section 7 of the reaction vessel 8 is pressurized and is forcibly fed as indicated by arrows A in Figure 3 from the upper side of the biochemical analysis unit 1 held at the biochemical analysis unit holding section 7 toward the lower side thereof so as to cut through a number of the absorptive regions 4 formed in the substrate 2 of the biochemical analysis unit 1.

As a result, a substance derived from a living organism and contained in the hybridization reaction solution is selectively hybridized with specific binding substances contained in a number of the absorptive regions 4 of the biochemical analysis unit 1.

The piston motor (not shown) is next driven for driving the piston 15 of the syringe 16 so that the piston 15 is moved downward in Figure 3.

When the piston 15 of the syringe 16 is moved downward in Figure 3, the hybridization reaction solution contained in the space below the biochemical analysis unit 1 held at the biochemical analysis unit holding section 7 of the reaction vessel 8 is pressurized and is forcibly fed as indicated by arrows B in Figure 3 from the lower side of the biochemical analysis unit 1 held at the biochemical analysis unit holding section 7 toward the upper side thereof so as to cut through a number of the absorptive regions 4 formed in the substrate 2 of the biochemical analysis unit 1.

As a result, a substance derived from a living organism and contained in the hybridization reaction solution is selectively hybridized with specific binding substances contained in a number of the absorptive regions 4 of the biochemical analysis unit 1.

When similar operations have been repeated for a predetermined time period, hybridization is completed.

In this manner, in this embodiment, since the hybridization reaction solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, it is possible to markedly increase the moving rate of a substance derived from a living organism and contained in the hybridization reaction solution through the absorptive regions 4 of the biochemical analysis unit 1 in comparison with the case of moving a substance derived from a living organism and contained in the hybridization reaction solution only by convection or diffusion and hybridizing specific binding substances. Therefore, since it is possible to markedly increase the reaction rate of hybridization and it is possible to markedly increase the possibility of association of the substance derived from a living organism and contained in the hybridization reaction solution with the specific binding substances contained in deep portions of a number of the absorptive regions 4 of the biochemical analysis unit 1, the substance derived from a living organism and contained in the hybridization reaction solution can be hybridized with the specific binding substances fixed in a number of the absorptive regions 4 of the biochemical analysis unit 1 in a desired manner.

When the hybridization reaction has been completed in this manner, the solution circulation pipe 14 is removed from the solution feeding opening 12 and the hybridization reaction solution is discharged from the reaction vessel 8.

When the discharge of the hybridization reaction solution from the reaction vessel 8 has been completed, a cleaning solution is fed into the reaction vessel 8 through the solution feeding opening 12.

When the inside of the reaction vessel 8 has been filled with the cleaning solution, the solution circulation pipe 14 is attached to the solution feeding opening 12.

The piston motor (not shown) is driven for driving the piston 15 of the syringe 16 so that the piston 15 is moved upward in Figure 3.

When the piston 15 of the syringe 16 is moved upward in Figure 3, the cleaning solution contained in the space above the biochemical analysis unit 1 held at the biochemical analysis unit holding section 7 of the reaction vessel 8 is pressurized and is forcibly fed as indicated by the arrows A in Figure 3 from the upper side of the biochemical analysis unit 1 held at the biochemical analysis unit holding section 7 toward the lower side thereof so as to cut through a number of the absorptive regions 4 formed in the substrate 2 of the biochemical analysis unit 1.

The piston motor (not shown) is next driven for driving the piston 15 of the syringe 16 so that the piston 15 is moved downward in Figure 3.

When the piston 15 of the syringe 16 is moved downward in Figure 3, the cleaning solution contained in the space below the biochemical analysis unit 1 held at the biochemical analysis unit holding section 7 of the reaction vessel 8 is pressurized and is forcibly fed as indicated by the arrows B in Figure 3 from the lower side of the biochemical analysis unit 1 held at the biochemical analysis unit holding section 7 toward the upper side thereof so as to cut through a number of the absorptive regions 4 formed in the substrate 2 of the biochemical analysis unit 1.

When similar operations have been repeated for a predetermined time period, the operation for cleaning a number of the absorptive regions 4 formed in the substrate 2 of the biochemical analysis unit 1 is completed.

In this manner, in this embodiment, since the cleaning solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, even if a substance derived from a living organism which should not be hybridized with specific binding substances absorbed in the absorptive regions 4 formed in the substrate 2 of the biochemical analysis unit 1 has been absorbed in the absorptive regions 4 during the process of hybridization, it is possible to efficiently peel off and remove the substance derived from a living organism which should not be hybridized with specific binding substances absorbed in the absorptive regions 4 from a number of the absorptive regions 4 of the biochemical analysis unit 1 and therefore, it is possible to markedly improve the efficiency of the cleaning operation.

When the cleaning operation has been completed in this manner, the solution circulation pipe 14 is removed from the solution feeding opening 12 and the cleaning solution is discharged from the reaction vessel 8.

As described above, radiation data of a radioactive labeling substance and a fluorescence data of a fluorescent substance such as a fluorescent dye are recorded in a number of the absorptive regions 4 formed in the substrate 2 of the biochemical analysis unit 1.

The fluorescence data recorded in a number of the absorptive regions 4 of the biochemical analysis unit 1 are read by a scanner described later and biochemical analysis data are produced.

On the other hand, radiation data recorded in a number of the absorptive regions 4 of the biochemical analysis unit 1 are transferred onto a stimulable phosphor sheet described later and read by a scanner described later, thereby producing biochemical analysis data.

To the contrary, in order to record chemiluminescence data in a number of the absorptive regions 4 formed in the substrate 2 of the biochemical analysis unit 1, an antibody solution containing an antibody to the hapten such as digoxigenin labeled with an enzyme which generates chemiluminescence emission when it contacts a chemiluminescent substrate is further prepared and fed into the reaction vessel 8 and the antibody to the hapten such as digoxigenin labeled with an enzyme which generates chemiluminescence emission when it contacts a chemiluminescent substrate is bound with the hapten such as digoxigenin labeling a substance derived from a living organism selectively hybridized with specific binding substances absorbed in a number of the absorptive regions 4 formed in the substrate 2 of the biochemical analysis unit 1 by the an antigen-antibody reaction.

Specifically, when the discharge of the cleaning solution from the reaction vessel 8 has been completed, an antibody solution containing an antibody to the hapten such as digoxigenin labeled with an enzyme which generates chemiluminescence emission when it contacts a chemiluminescent substrate is first prepared and is fed into the reaction vessel 8 through the solution feeding opening 12.

When the inside of the reaction vessel 8 has been filled with the antibody solution, the solution circulation pipe 14 is attached to the solution feeding opening 12.

The piston motor (not shown) is driven for driving the piston 15 of the syringe 16 so that the piston 15 is moved upward in Figure 3.

When the piston 15 of the syringe 16 is moved upward in Figure 3, the antibody solution contained in the space above the biochemical analysis unit 1 held at the biochemical analysis unit holding section 7 of the reaction vessel 8 is pressurized and is forcibly fed as indicated by the arrows A in Figure 3 from the upper side of the biochemical analysis unit 1 held at the biochemical analysis unit holding section 7 toward the lower side thereof so as to cut through a number of the absorptive regions 4 formed in the substrate 2 of the biochemical analysis unit 1, whereby an antibody to the hapten labeled with an enzyme which generates chemiluminescence emission when it contacts a chemiluminescent substrate is bonded by an antigen-antibody reaction with a hapten labeling a substance derived from a living organism and selectively hybridized with specific biding substances absorbed in a number of the absorptive regions 4 formed in the substrate 2 of the biochemical analysis unit 1.

The piston motor (not shown) is next driven for driving the piston 15 of the syringe 16 so that the piston 15 is moved downward in Figure 3.

When the piston 15 of the syringe 16 is moved downward in Figure 3, the antibody solution contained in the space below the biochemical analysis unit 1 held at the biochemical analysis unit holding section 7 of the reaction vessel 8 is pressurized and is forcibly fed as indicated by the arrows B in Figure 3 from the lower side of the biochemical analysis unit 1 held at the biochemical analysis unit holding section 7 toward the upper side thereof so as to cut through a number of the absorptive regions 4 formed in the substrate 2 of the biochemical analysis unit 1, whereby an antibody to the hapten labeled with an enzyme which generates chemiluminescence emission when it contacts a chemiluminescent substrate is bonded by an antigen-antibody reaction with a hapten labeling a substance derived from a living organism and selectively hybridized with specific biding substances absorbed in a number of the absorptive regions 4 formed in the substrate 2 of the biochemical analysis unit 1.

When similar operations have been repeated for a predetermined time period, the antigen-antibody reaction of a hapten labeling a substance derived from a living organism and selectively hybridized with specific biding substances absorbed in a number of the absorptive regions 4 formed in the substrate 2 of the biochemical analysis unit 1 and an antibody to the hapten labeled with an enzyme which generates chemiluminescence emission when it contacts a chemiluminescent substrate is completed.

In this manner, in this embodiment, since the antibody solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, it is possible to markedly increase the moving rate of an antibody contained in the antibody solution through the absorptive regions 4 of the biochemical analysis unit 1 in comparison with the case of moving an antibody contained in the antibody solution only by convection or diffusion and binding a hapten. Therefore, since it is possible to markedly increase the reaction rate of an antigen-antibody reaction and it is possible to markedly increase the possibility of association of an antibody to a hapten contained in the antibody solution with the hapten labeling a substance derived from a living organism and selectively hybridized with specific binding substances contained in deep portions of a number of the absorptive regions 4 of the biochemical analysis unit 1, the antibody to a hapten contained in the antibody solution can be bound by an antigen-antibody reaction in a desired manner with the labeling a substance derived from a living organism and selectively hybridized with specific binding substances contained in the absorptive regions 4 of the biochemical analysis unit 1.

When the antigen-antibody reaction has been completed in this manner, the solution circulation pipe 14 is removed from the solution feeding opening 12 and the antibody solution is discharged from the reaction vessel 8.

When the discharge of the antibody solution from the reaction vessel 8 has been completed, a cleaning solution is fed into the reaction vessel 8 through the solution feeding opening 12.

When the inside of the reaction vessel 8 has been filled with the cleaning solution, the solution circulation pipe 14 is attached to the solution feeding opening 12 and similarly to the above, the piston 15 of the syringe 16 is moved vertically, thereby cleaning a number of the absorptive regions 4 formed in the substrate 2 of the biochemical analysis unit 1 with the cleaning solution.

Therefore, since the cleaning solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, even if an antibody which should not be bonded with the hapten labeling a substance derived from a living body and selectively hybridized with specific binding substances absorbed in the absorptive regions 4 of the biochemical analysis unit 1 has been absorbed in the absorptive regions 4 formed in the substrate 2 of the biochemical analysis unit 1 during the process of the antigen-antibody reaction, it is possible to efficiently peel off and remove the antibody which should not be bonded with the hapten from a number of the absorptive regions 4 of the biochemical analysis unit 1 and the efficiency of the cleaning operation can be markedly improved.

When the cleaning operation has been completed in this manner, the solution circulation pipe 14 is removed from the solution feeding opening 12 and the cleaning solution is discharged from the reaction vessel 8.

In this manner, chemiluminescent data are recorded in a number of the absorptive regions 4 formed in the substrate 2 of the biochemical analysis unit 1.

The chemiluminescent data recorded in a number of the absorptive regions 4 formed in the substrate 2 of the biochemical analysis unit 1 are read by a cooled CCD camera of a data producing system described later and biochemical analysis data are produced.

When the discharge of the cleaning solution from the reaction vessel 8 has been completed, the upper half portion 11 is removed from the main body 9 of the reaction vessel 8 and the biochemical analysis unit 1 held at the biochemical analysis unit holding section 7 is lifted from the reaction vessel 8 by the user.

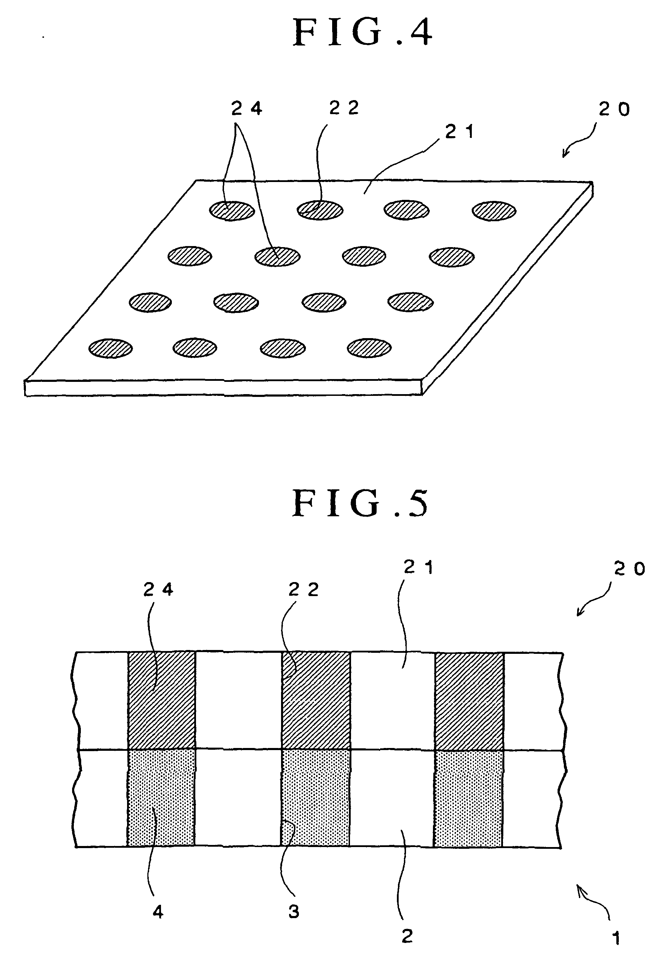

Figure 4 is a schematic perspective view showing a stimulable phosphor sheet.

As shown in Figure 4, a stimulable phosphor sheet 20 includes a support 21 made of nickel and regularly formed with a number of substantially circular through-holes 22 and a number of stimulable phosphor layer regions 24 are dot-like formed by embedding stimulable phosphor in a number of the through-holes 22 formed in the support 21.

A number of the through-holes 22 are formed in the support 21 in the same pattern as that of a number of the absorptive regions 4 formed in the substrate 2 of the biochemical analysis unit 1 and each of a number of the stimulable phosphor layer regions 24 has the same size as that of each of a number of the absorptive regions 4 formed in the substrate 2 of the biochemical analysis unit 1.

Therefore, although not accurately shown in Figure 4, the 19,200 substantially circular stimulable phosphor layer regions 24 having a size of about 0.01 mm² are formed the same regular pattern as that of a number of the absorptive regions 4 formed in the substrate 2 of the biochemical analysis unit 1 and in the manner of a matrix in the support 21 of the stimulable phosphor sheet 20.

In this embodiment, the stimulable phosphor sheet 20 is formed by embedding stimulable phosphor in a number of the through-holes 22 formed in the support 21 in such a manner that the surface of the support 21 and the surface of each of the stimulable phosphor layer regions 24 are located at the same height level.

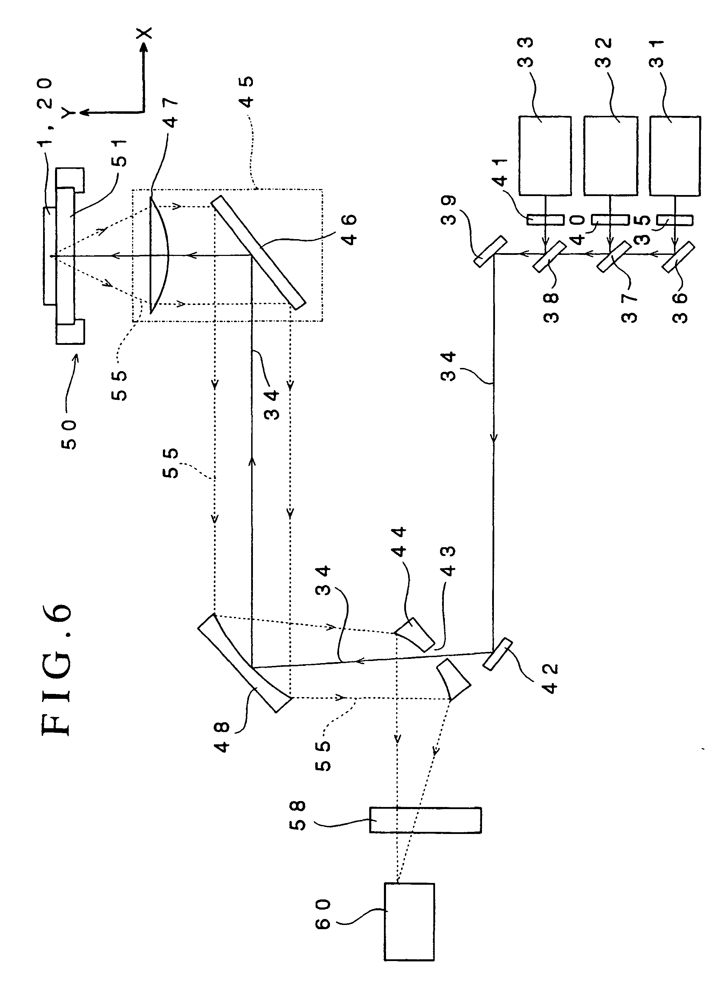

Figure 5 is a schematic cross-sectional view showing a method for exposing a number of the stimulable phosphor layer regions 24 formed in the stimulable phosphor sheet 20 to a radioactive labeling substance contained in a number of the absorptive regions 4 formed in the biochemical analysis unit 1.

As shown in Figure 5, when the stimulable phosphor layer regions 24 of the stimulable phosphor sheet 20 are to be exposed, the stimulable phosphor sheet 20 is superposed on the biochemical analysis unit 1 in such a manner that a number of the absorptive regions 4 formed in the biochemical analysis unit 1 face the corresponding stimulable phosphor layer regions 24 formed in the support 21 of the stimulable phosphor sheet 20.

In this manner, each of a number of the stimulable phosphor layer regions 24 formed in the support 21 of the stimulable phosphor sheet 20 is kept to face the corresponding absorptive region 4 formed in the biochemical analysis unit 1 for a predetermined time period, whereby a number of the stimulable phosphor layer regions 24 formed in the stimulable phosphor sheet 20 are exposed to the radioactive labeling substance selectively contained in a number of the absorptive regions 4 formed in the biochemical analysis unit 1.

During the exposure operation, electron beams (B rays) are released from the radioactive labeling substance absorbed in the absorptive regions 4 of the biochemical analysis unit 1. However, since a number of the absorptive regions 4 of the biochemical analysis unit 1 are formed so as to be spaced from each other in the substrate 2 made of stainless steel having a property of attenuating radiation energy, electron beams (B rays) released from a particular absorptive region 4 of the biochemical analysis unit 1 can be efficiently prevented from scattering in the substrate 2 of the biochemical analysis unit 1, thereby mixing with electron beams (B rays) released from neighboring absorptive regions 4 and entering stimulable phosphor layer regions 24 next the stimulable phosphor layer region 24 corresponding thereto. Further, since a number of the stimulable phosphor layer regions 24 of the stimulable phosphor sheet 20 are formed by embedding stimulable phosphor in a number of the through-holes 22 formed in the support 21 made of nickel and the support 21 is capable of attenuating radiation energy, electron beams (B rays) released from the absorptive regions 4 of the biochemical analysis unit 1 can be efficiently prevented from scattering in the support 21 of the stimulable phosphor sheet 20 and entering stimulable phosphor layer regions 24 next to the corresponding stimulable phosphor layer region 24. Therefore, since it is possible to selectively impinge electron beams (B rays) released from the radioactive labeling substance contained in the individual absorptive regions 4 onto the corresponding stimulable phosphor layer regions 24, it is possible to reliably prevent electron beams (β rays) released from the radioactive labeling substance contained in the individual absorptive regions 4 from entering the stimulable phosphor layer regions 24 of the stimulable phosphor sheet 20 to be exposed to electron beams (β rays) released from neighboring absorptive regions 4 and exposing stimulable phosphor contained therein.

In this manner, radiation data of a radioactive labeling substance are recorded in a number of the stimulable phosphor layer regions 24 formed in the support 21 of the stimulable phosphor sheet 20.

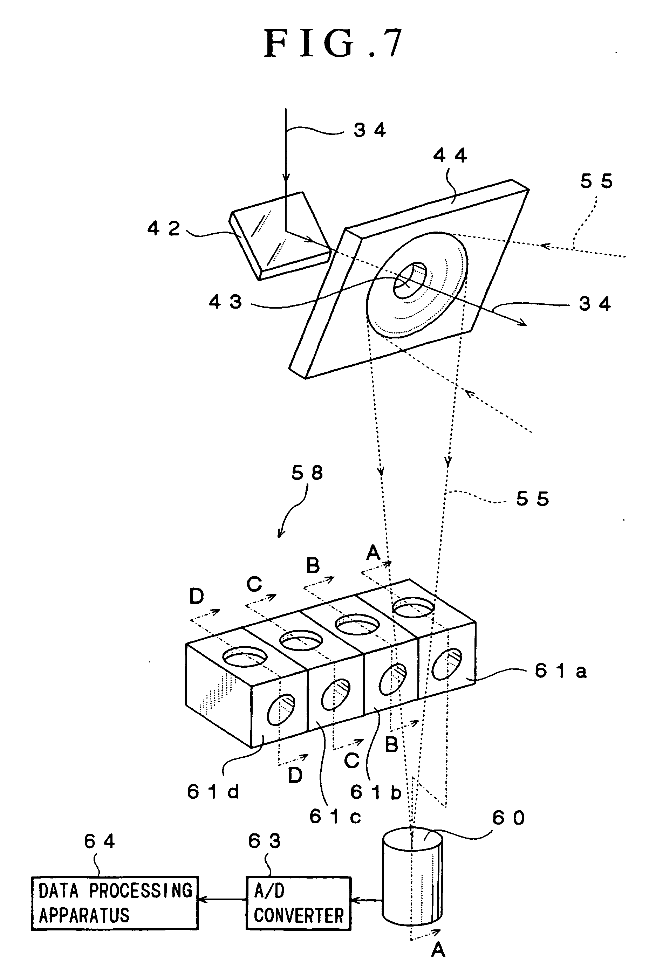

Figure 6 is a schematic view showing a scanner for reading radiation data of a radioactive labeling substance recorded in a number of the stimulable phosphor layer regions 24 formed in the support 21 of the stimulable phosphor sheet 20 and fluorescence data of a fluorescent substance such as a fluorescent dye recorded in a number of the absorptive regions 4 formed in the substrate 2 of the biochemical analysis unit 1 and producing biochemical analysis data, and Figure 7 is a schematic perspective view showing details in the vicinity of a photomultiplier of the scanner.

The scanner shown according to this embodiment is constituted so as to read radiation data of a radioactive labeling substance recorded in a number of the stimulable phosphor layer regions 24 formed in the stimulable phosphor sheet 20 and fluorescence data of a fluorescent substance such as a fluorescent dye recorded in a number of the absorptive regions 4 of the biochemical analysis unit 1 to produce biochemical analysis data and includes a first laser stimulating ray source 31 for emitting a laser beam 34 having a wavelength of 640 nm, a second laser stimulating ray source 32 for emitting a laser beam 34 having a wavelength of 532 nm and a third laser stimulating ray source 33 for emitting a laser beam 34 having a wavelength of 473 nm.

In this embodiment, the first laser stimulating ray source 31 is constituted by a semiconductor laser beam source and the second laser stimulating ray source 32 and the third laser stimulating ray source 33 are constituted by a second harmonic generation element.

A laser beam 34 emitted from the first laser stimulating source 31 passes through a collimator lens 35, thereby being made a parallel beam, and is reflected by a mirror 36. A first dichroic mirror 37 for transmitting light having a wavelength of 640 nm but reflecting light having a wavelength of 532 nm and a second dichroic mirror 38 for transmitting light having a wavelength equal to and longer than 532 nm but reflecting light having a wavelength of 473 nm are provided in the optical path of the laser beam 34 emitted from the first laser stimulating ray source 31. The laser beam 34 emitted from the first laser stimulating ray source 31 and reflected by the mirror 36 passes through the first dichroic mirror 37 and the second dichroic mirror 38 and advances to a mirror 39.

On the other hand, the laser beam 34 emitted from the second laser stimulating ray source 32 passes through a collimator lens 40, thereby being made a parallel beam, and is reflected by the first dichroic mirror 37, thereby changing its direction by 90 degrees. The laser beam 34 then passes through the second dichroic mirror 38 and advances to the mirror 39.

Further, the laser beam 34 emitted from the third laser stimulating ray source 33 passes through a collimator lens 41, thereby being made a parallel beam, and is reflected by the second dichroic mirror 38, thereby changing its direction by 90 degrees. The laser beam 34 then advances to the mirror 39.

The laser beam 34 advancing to the mirror 39 is reflected by the mirror 39 and advances to a mirror 42 to be reflected thereby.

A perforated mirror 44 formed with a hole 43 at the center portion thereof is provided in the optical path of the laser beam 34 reflected by the mirror 42. The laser beam 34 reflected by the mirror 42 passes through the hole 43 of the perforated mirror 44 and advances to a concave mirror 48.

The laser beam 34 advancing to the concave mirror 48 is reflected by the concave mirror 48 and enters an optical head 45.

The optical head 45 includes a mirror 46 and an aspherical lens 47. The laser beam 34 entering the optical head 45 is reflected by the mirror 46 and impinged by the aspherical lens 47 onto one of a number of the stimulable phosphor layer regions 24 of the stimulable phosphor sheet 20 or one of a number of the absorptive regions 4 of the biochemical analysis unit 1 placed on the glass plate 51 of a stage 50.

When the laser beam 34 impinges on one of the stimulable phosphor layer regions 24 formed in the support 21 of the stimulable phosphor sheet 20, stimulable phosphor contained in the stimulable phosphor layer region 24 is excited, thereby releasing stimulated emission 55. On the other hand, when the laser beam 34 impinges on one of the absorptive regions 4 formed in the substrate 2 of the biochemical analysis unit 1, a fluorescent substance such as a fluorescent dye contained in the absorptive region 4 is excited, thereby releasing fluorescence emission 55.

The stimulated emission 55 released from the stimulable phosphor layer region 24 formed in the stimulable phosphor 20 or the fluorescence emission 55 released from the absorptive region 4 formed in the biochemical analysis unit 1 is condensed onto the mirror 46 by the aspherical lens 47 provided in the optical head 45 and reflected by the mirror 46 on the side of the optical path of the laser beam 34, thereby being made a parallel beam to advance to the concave mirror 48.

The stimulated emission 55 or the fluorescence emission 55 advancing to the concave mirror 48 is reflected by the concave mirror 48 and advances to the perforated mirror 44.

As shown in Figure 7, the stimulated emission 55 or the fluorescence emission 55 advancing to the perforated mirror 44 is reflected downward by the perforated mirror 44 formed as a concave mirror and advances to a filter unit 58, whereby light having a predetermined wavelength is cut. The stimulated emission 55 or the fluorescence emission 55 then impinges on a photomultiplier 60, thereby being photoelectrically detected.

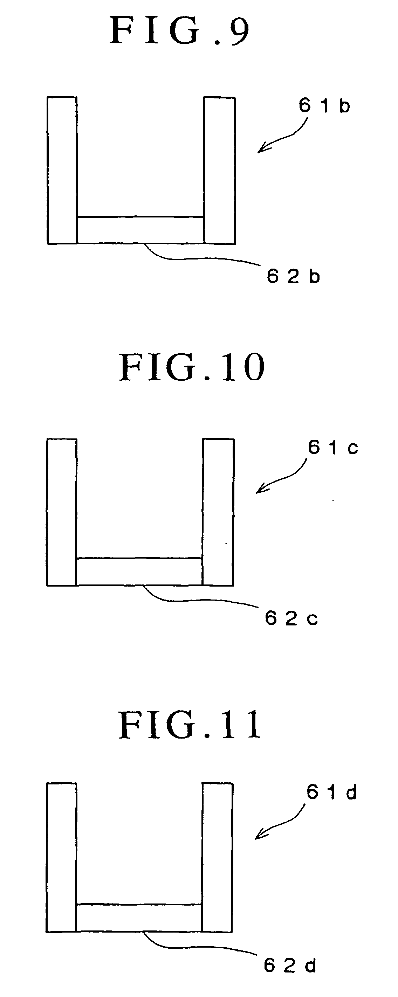

As shown in Figure 7, the filter unit 58 is provided with four filter members 61a, 61b, 61c and 61d and is constituted to be laterally movable in Figure 10 by a motor (not shown).

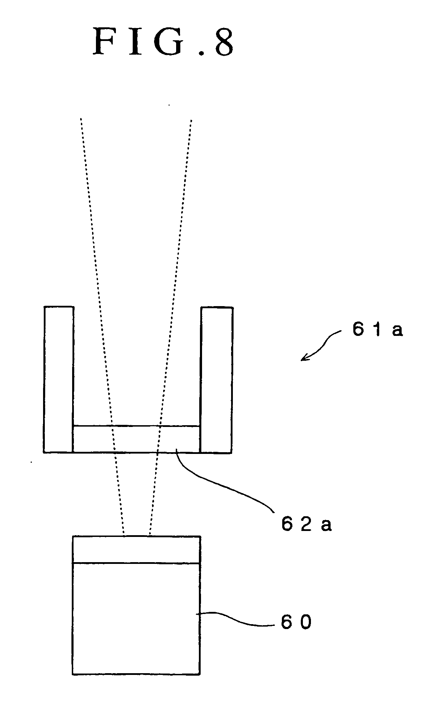

Figure 8 is a schematic cross-sectional view taken along a line A-A in Figure 7.

As shown in Figure 8, the filter member 61a includes a filter 62a and the filter 62a is used for reading fluorescence emission 55 by stimulating a fluorescent substance such as a fluorescent dye contained in a number of the absorptive regions 4 formed in the biochemical analysis unit 1 using the first laser stimulating ray source 31 and has a property of cutting off light having a wavelength of 640 nm but transmitting light having a wavelength longer than 640 nm.

Figure 9 is a schematic cross-sectional view taken along a line B-B in Figure 7.

As shown in Figure 9, the filter member 61b includes a filter 62b and the filter 62b is used for reading fluorescence emission 55 by stimulating a fluorescent substance such as a fluorescent dye contained in a number of the absorptive regions 4 formed in the biochemical analysis unit 1 using the second laser stimulating ray source 32 and has a property of cutting off light having a wavelength of 532 nm but transmitting light having a wavelength longer than 532 nm.

Figure 10 is a schematic cross-sectional view taken along a line C-C in Figure 7.

As shown in Figure 10, the filter member 61c includes a filter 62c and the filter 62c is used for reading fluorescence emission 55 by stimulating a fluorescent substance such as a fluorescent dye contained in a number of the absorptive regions 4 formed in the biochemical analysis unit 1 using the third laser stimulating ray source 33 and has a property of cutting off light having a wavelength of 473 nm but transmitting light having a wavelength longer than 473 nm.

Figure 11 is a schematic cross-sectional view taken along a line D-D in Figure 7.

As shown in Figure 11, the filter member 61d includes a filter 62d and the filter 62d is used for reading stimulated emission 55 released from stimulable phosphor contained in a number of the stimulable phosphor layer regions 24 formed in the stimulable phosphor sheet 20 upon being stimulated using the first laser stimulating ray source 31 and has a property of transmitting only light having a wavelength corresponding to that of stimulated emission 55 emitted from stimulable phosphor and cutting off light having a wavelength of 640 nm.

Therefore, in accordance with the kind of a stimulating ray source to be used, one of these filter members 61a, 61b, 61c, 61d is selectively positioned in front of the photomultiplier 60, thereby enabling the photomultiplier 60 to photoelectrically detect only light to be detected.

The analog data produced by photoelectrically detecting stimulated emission 55 or fluorescence emission 55 with the photomultiplier 60 are converted by an A/D converter 63 into digital data and the digital data are fed to a data processing apparatus 64.

Although not shown in Figure 6, the optical head 45 is constituted to be movable by a scanning mechanism in a main scanning direction indicated by an arrow X and a sub-scanning direction indicated by an arrow Y in Figure 6 so that all of the stimulable phosphor layer regions 24 formed in the support 21 of the stimulable phosphor sheet 20 or all of the absorptive regions 4 formed in the substrate 2 of the biochemical analysis unit 1 can be scanned by the laser beam 34.

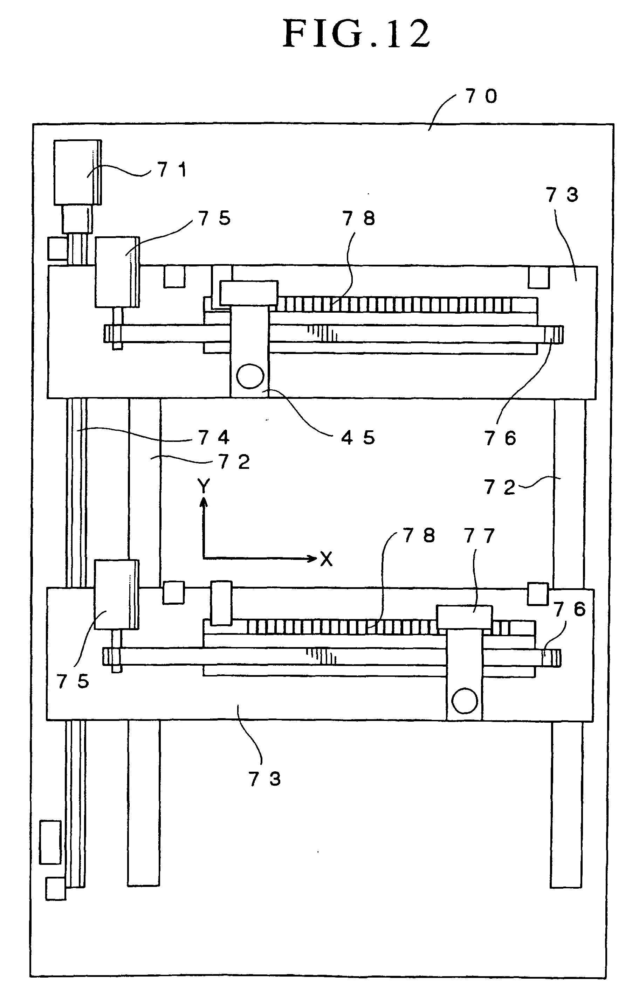

Figure 12 is a schematic plan view showing the scanning mechanism of the optical head 45.

In Figure 12, optical systems other than the optical head 45 and the paths of the laser beam 34 and stimulated emission 55 or fluorescence emission 55 are omitted for simplification.

As shown in Figure 12, the scanning mechanism of the optical head 45 includes a base plate 70, and a sub-scanning pulse motor 71 and a pair of rails 72, 72 are fixed on the base plate 70. A movable base plate 73 is further provided so as to be movable in the sub-scanning direction indicated by an arrow Y in Figure 12.

The movable base plate 73 is formed with a threaded hole (not shown) and a threaded rod 74 rotated by the sub-scanning pulse motor 71 is engaged with the inside of the hole.

A main scanning stepping motor 75 is provided on the movable base plate 73. The main scanning stepping motor 75 is adapted for intermittently driving an endless belt 76 by a pitch equal to the distance between neighboring absorptive regions 4 formed in the substrate 2 of the biochemical analysis unit 1, namely, the distance between neighboring stimulable phosphor layer regions 24 formed in the support 21 of the stimulable phosphor sheet 20. The optical head 45 is fixed to the endless belt 76 and when the endless belt 76 is driven by the main scanning stepping motor 75, the optical head 45 is moved in the main scanning direction indicated by an arrow X in Figure 12.

In Figure 12, the reference numeral 77 designates a linear encoder for detecting the position of the optical head 45 in the main scanning direction and the reference numeral 78 designates slits of the linear encoder 77.

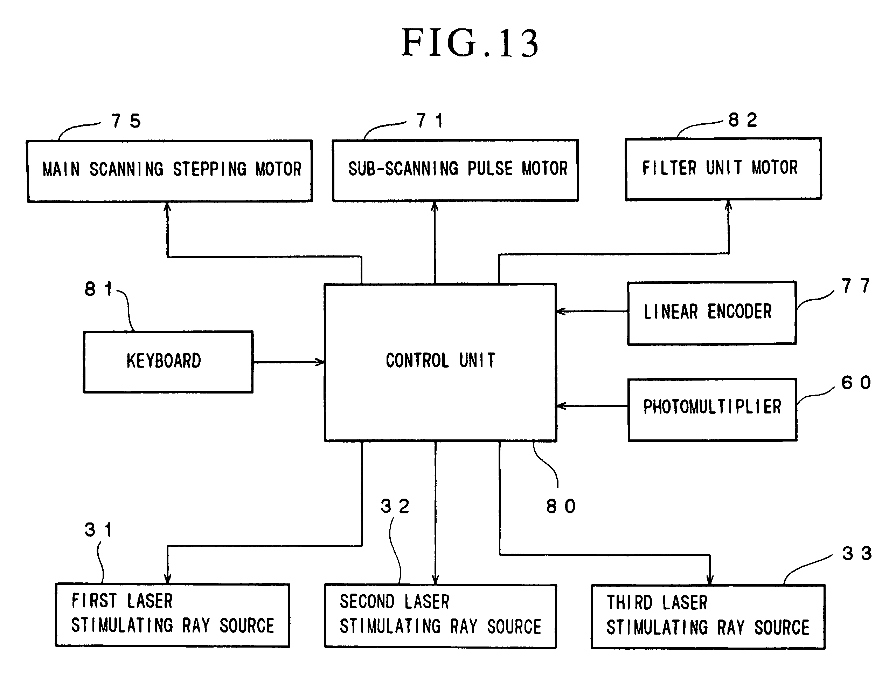

Therefore, when the endless belt 76 is driven in the main scanning direction by the main scanning stepping motor 75 and the scanning of one line is completed, the substrate 73 is intermittently moved in the sub-scanning direction by the sub-scanning pulse motor 71, whereby the optical head 45 is moved in the main scanning direction indicated by the arrow X and the sub-scanning direction indicated by the arrow Y in Figure 13 and all of the stimulable phosphor layer regions 24 formed in the support 21 of the stimulable phosphor sheet 24 or all of the absorptive regions 4 formed in the substrate 2 of the biochemical analysis unit 1 are scanned with the laser beam 34.

Figure 13 is a block diagram of a control system, an input system, a drive system and a detection system of the scanner shown in Figure 6.

As shown in Figure 13, the control system of the scanner includes a control unit 80 for controlling the overall operation of the scanner and the input system of the scanner includes a keyboard 81 which can be operated by a user and through which various instruction signals can be input.

As shown in Figure 13, the drive system of the scanner includes the main scanning stepping motor 75 for intermittently moving the optical head 45 in the main scanning direction, the sub-scanning pulse motor 71 for moving the optical head 45 in the sub-scanning direction and a filter unit motor 82 for moving the filter unit 58 provided with the four filter members 61a, 61b, 61c and 61d.

The control unit 80 is adapted for selectively outputting a drive signal to the first laser stimulating ray source 31, the second laser stimulating ray source 32 or the third laser stimulating ray source 33 and outputting a drive signal to the filter unit motor 82.

As shown in Figure 13, the detection system of the scanner includes the photomultiplier 60 and the linear encoder 77 for detecting the position of the optical head 45 in the main scanning direction.

In this embodiment, the control unit 80 is adapted to control the on and off operation of the first laser stimulating ray source 31, the second laser stimulating ray source 32 or the third laser stimulating ray source 33 in accordance with a detection signal indicating the position of the optical head 45 input from the linear encoder 77.

The thus constituted scanner reads radiation data of a radioactive labeling substance recorded in a stimulable phosphor sheet 20 by exposing a number of the stimulable phosphor layer regions 24 to a radioactive labeling substance contained in a number of the absorptive regions 4 formed in the substrate 2 of the biochemical analysis unit 1 and produces biochemical analysis data in the following manner.

A stimulable phosphor sheet 20 is first set on the glass plate 51 of the stage 50 by a user.

An instruction signal indicating that a number of the stimulable phosphor regions 24 formed in the support 21 of the stimulable phosphor sheet 20 are to be scanned with the laser beam 34 is then input through the keyboard 81.

The instruction signal input through the keyboard 81 is input to the control unit 80 and the control unit 80 outputs a drive signal to the filter unit motor 82 in accordance with the instruction signal, thereby moving the filter unit 58 so as to locate the filter member 61d provided with the filter 62d having a property of transmitting only light having a wavelength corresponding to that of stimulated emission emitted from stimulable phosphor but cutting off light having a wavelength of 640 nm in the optical path of stimulated emission 55.

The control unit 80 further outputs a drive signal to the main scanning stepping motor 75 to move the optical head 45 in the main scanning direction and when it determines based on a detection signal indicating the position of the optical head 45 input from the linear encoder 77 that the optical head 45 has reached a position where a laser beam 34 can be projected onto a first stimulable phosphor layer region 24 among a number of the stimulable phosphor layer regions 24 formed in the support 21 of stimulable phosphor sheet 20, it outputs a drive stop signal to the main scanning stepping motor 75 and a drive signal to the first stimulating ray source 31, thereby actuating it to emit a laser beam 34 having a wavelength of 640 nm.

A laser beam 34 emitted from the first laser stimulating source 31 passes through the collimator lens 35, thereby being made a parallel beam, and is reflected by the mirror 36.

The laser beam 34 reflected by the mirror 36 passes through the first dichroic mirror 37 and the second dichroic mirror 38 and advances to the mirror 39.

The laser beam 34 advancing to the mirror 39 is reflected by the mirror 39 and advances to the mirror 42 to be reflected thereby.

The laser beam 34 reflected by the mirror 42 passes through the hole 43 of the perforated mirror 44 and advances to the concave mirror 48.

The laser beam 34 advancing to the concave mirror 48 is reflected by the concave mirror 48 and enters the optical head 45.

The laser beam 34 entering the optical head 45 is reflected by the mirror 46 and condensed by the aspherical lens 47 onto the first stimulable phosphor layer region 24 formed in the support 21 of the stimulable phosphor sheet 20 placed on the glass plate 51 of a stage 50.

In this embodiment, since a number of the stimulable phosphor layer regions 24 of the stimulable phosphor sheet 20 are formed by embedding stimulable phosphor in a number of the through-holes 22 formed in the support 21 made of nickel capable of attenuating light energy, it is possible to effectively prevent the laser beam 24 from scattering in each of the stimulable phosphor layer regions 24 formed in the support 21 of the stimulable phosphor sheet 20 and entering the neighboring stimulable phosphor layer regions 24 to excite stimulable phosphor contained in the neighboring stimulable phosphor layer regions 24.

When the laser beam 34 impinges onto the first stimulable phosphor layer region 24 formed in the support 21 of the stimulable phosphor sheet 20, stimulable phosphor contained in the first stimulable phosphor layer region 24 is excited by the laser beam 34, thereby releasing stimulated emission 55 from the first stimulable phosphor layer region 24.

The stimulated emission 55 released from the first stimulable phosphor layer region 24 of the stimulable phosphor sheet 20 is condensed onto the mirror 46 by the aspherical lens 47 provided in the optical head 45 and reflected by the mirror 46 on the side of the optical path of the laser beam 34, thereby being made a parallel beam to advance to the concave mirror 48.

The stimulated emission 55 advancing to the concave mirror 48 is reflected by the concave mirror 48 and advances to the perforated mirror 44.

As shown in Figure 7, the stimulated emission 55 advancing to the perforated mirror 44 is reflected downward by the perforated mirror 44 formed as a concave mirror and advances to the filter 62d of the filter unit 58.

Since the filter 62d has a property of transmitting only light having a wavelength corresponding to that of stimulated emission 55 emitted from stimulable phosphor and cutting off light having a wavelength of 640 nm, light having a wavelength of 640 nm corresponding to that of the stimulating ray is cut off by the filter 62d and only light having a wavelength corresponding to that of stimulated emission 55 passes through the filter 62d to be photoelectrically detected by the photomultiplier 60.

Analog data produced by photoelectrically detecting stimulated emission 55 with the photomultiplier 60 are converted by an A/D converter 63 into digital data and the digital data are fed to a data processing apparatus 64.

When a predetermined time, for example, several microseconds, has passed after the first stimulating ray source 31 was turned on, the control unit 80 outputs a drive stop signal to the first stimulating ray source 31, thereby turning it off and outputs a drive signal to the main scanning stepping motor 75, thereby moving the optical head 45 by one pitch equal to the distance between neighboring stimulable phosphor layer regions 24 formed in the support 21 of the stimulable phosphor sheet 20.

When the control unit 80 determines based on a detection signal indicating the position of the optical head 45 input from the linear encoder 77 that the optical head 45 has been moved by one pitch equal to the distance between neighboring stimulable phosphor layer regions 24 and has reached a position where a laser beam 34 can be projected onto a second stimulable phosphor layer region 24 next to the first stimulable phosphor layer region 24 formed in the support 21 of the stimulable phosphor sheet 20, it outputs a drive signal to the first stimulating ray source 31 to turn it on, thereby causing the laser beam 34 to excite stimulable phosphor contained in the second stimulable phosphor layer region 24 formed in the support 21 of the stimulable phosphor sheet 20 next to the first stimulable phosphor layer region 24.

Similarly to the above, the second stimulable phosphor layer region 24 formed in the support 21 of the stimulable phosphor sheet 20 is irradiated with the laser beam 34 for a predetermined time, whereby stimulable phosphor contained in the second stimulable phosphor layer region 24 is excited and when stimulated emission 55 released from the second stimulable phosphor layer region 24 is photoelectrically detected by the photomultiplier 60 and analog data are produced, the control unit 80 outputs a drive stop signal to the first stimulating ray source 31, thereby turning it off and outputs a drive signal to the main scanning stepping motor 75, thereby moving the optical head 45 by one pitch equal to the distance between neighboring stimulable phosphor layer regions 24.

In this manner, the on and off operation of the first stimulating ray source 31 is repeated in synchronism with the intermittent movement of the optical head 45 and when the control unit 80 determines based on a detection signal indicating the position of the optical head 45 input from the linear encoder 77 that the optical head 45 has been moved by one scanning line in the main scanning direction and that the stimulable phosphor layer regions 24 included in a first line of the stimulable phosphor layer regions 24 formed in the support 21 of the stimulable phosphor sheet 20 have been scanned with the laser beam 34, it outputs a drive signal to the main scanning stepping motor 75, thereby returning the optical head 45 to its original position and outputs a drive signal to the sub-scanning pulse motor 71, thereby causing it to move the movable base plate 73 by one scanning line in the sub-scanning direction.

When the control unit 80 determines based on a detection signal indicating the position of the optical head 45 input from the linear encoder 77 that the optical head 45 has been returned to its original position and determines that the movable base plate 73 has been moved by one scanning line in the sub-scanning direction, similarly to the manner in which the stimulable phosphor layer regions 24 included in the first line of the stimulable phosphor layer regions 24 formed in the support 21 of the stimulable phosphor sheet 20 were sequentially irradiated with the laser beam 34 emitted from the first laser stimulating ray source 31, the stimulable phosphor layer regions 24 included in a second line of the stimulable phosphor layer regions 24 formed in the support 21 of the stimulable phosphor sheet 20 are sequentially irradiated with the laser beam 34 emitted from the first laser stimulating ray source 31, thereby exciting stimulable phosphor contained in the stimulable phosphor layer regions 24 included in the second line and stimulated emission 55 released from the stimulable phosphor layer regions 24 included in the second line is sequentially and photoelectrically detected by the photomultiplier 60.

Analog data produced by photoelectrically detecting stimulated emission 55 with the photomultiplier 60 are converted by an A/D converter 63 into digital data and the digital data are fed to a data processing apparatus 64.

When all of the stimulable phosphor layer regions 24 formed in the support 21 of the stimulable phosphor sheet 20 have been scanned with the laser beam 34 to excite stimulable phosphor contained in the stimulable phosphor layer regions 24 and digital data produced by photoelectrically detecting stimulated emission 55 released from the stimulable phosphor layer regions 24 by the photomultiplier 60 to produce analog data and digitizing the analog data by the A/D converter 63 have been forwarded to the data processing apparatus 64, the control unit 80 outputs a drive stop signal to the first laser stimulating ray source 31, thereby turning it off.

As described above, radiation data recorded in a number of the stimulable phosphor layer regions 24 formed in the support 21 of the stimulable phosphor sheet 20 are read by the scanner and biochemical analysis data are produced.

On the other hand, when fluorescence data of a fluorescent substance recorded in a number of the absorptive regions 4 formed in the substrate 2 of the biochemical analysis unit 1 are to be read to produce biochemical analysis data, the biochemical analysis unit 1 is first set by the user on the glass plate 51 of the stage 50.

A fluorescent substance identification signal for identifying the kind of a fluorescent substance used as a labeling substance and a reading instruction signal indicating that fluorescent data are to be read are then input by the user through the keyboard 81.

The fluorescent substance identification signal and the instruction signal input through the keyboard 81 are input to the control unit 80 and when the control unit 80 receives them, it determines the laser stimulating ray source to be used in accordance with a table stored in a memory (not shown) and also determines what filter is to be positioned in the optical path of fluorescence emission 55 among the filters 62a, 62b and 62c.

For example, when Rhodamine (registered trademark), which can be most efficiently stimulated by a laser beam having a wavelength of 532 nm, is used as a fluorescent substance for labeling a substance derived from a living organism and the fluorescent substance identification signal indicating such a fact is input, the control unit 80 selects the second laser stimulating ray source 32 and the filter 62b and outputs a drive signal to the filter unit motor 82, thereby moving the filter unit 58 so that the filter member 61b inserting the filter 62b having a property of cutting off light having a wavelength of 532 nm but transmitting light having a wavelength longer than 532 nm in the optical path of the fluorescence emission 55.

The control unit 80 further outputs a drive signal to the main scanning stepping motor 75 to move the optical head 45 in the main scanning direction and when it determines based on a detection signal indicating the position of the optical head 45 input from the linear encoder 77 that the optical head 45 has reached a position where a laser beam 34 can be projected onto a first absorptive region 4 among a number of the absorptive regions 4 formed in the substrate 2 of the biochemical analysis unit 1, it outputs a drive stop signal to the main scanning stepping motor 75 and a drive signal to the second laser stimulating ray source 32, thereby actuating it to emit a laser beam 34 having a wavelength of 532 nm.

The laser beam 34 emitted from the second laser stimulating ray source 32 is made a parallel beam by the collimator lens 40, advances to the first dichroic mirror 37 and is reflected thereby.

The laser beam 34 reflected by the first dichroic mirror 37 transmits through the second dichroic mirror 38 and advances to the mirror 39.

The laser beam 34 advancing to the mirror 39 is reflected by the mirror 39 and further advances to the mirror 42 to be reflected thereby.

The laser beam 34 reflected by the mirror 42 advances to the perforated mirror 44 and passes through the hole 43 of the perforated mirror 44. Then, the laser beam 34 advances to the concave mirror 48.

The laser beam 34 advancing to the concave mirror 48 is reflected thereby and enters the optical head 45.

The laser beam 34 entering the optical head 45 is reflected by the mirror 46 and condensed by the aspherical lens 47 onto the first absorptive region 4 of the biochemical analysis unit 1 placed on the glass plate 51 of the stage 50.

In this embodiment, since each of the absorptive regions 4 of the biochemical analysis unit 1 is formed by charging nylon-6, 6 in the through-hole 3 formed in the substrate 2 made of stainless steel and the substrate 2 is capable of attenuating light energy, it is possible to effectively prevent the laser beam 24 from scattering in each of the absorptive regions 4 formed in the substrate 2 of the biochemical analysis unit 1 and entering the neighboring absorptive regions 4 to excite a fluorescent substance contained in the neighboring absorptive regions 4.

When the laser beam 34 impinges onto the first absorptive region 4 formed in the substrate 2 of the biochemical analysis unit 1, a fluorescent substance such as a fluorescent dye, for instance, Rhodamine, contained in the first absorptive region 4 is stimulated by the laser beam 34 and fluorescence emission 55 is released from Rhodamine.

The fluorescence emission 55 released from Rhodamine is condensed by the aspherical lens 47 provided in the optical head 45 and reflected by the mirror 46 on the side of an optical path of the laser beam 34, thereby being made a parallel beam to advance to the concave mirror 48.

The fluorescence emission 55 advancing to the concave mirror 48 is reflected by the concave mirror 48 and advances to the perforated mirror 44.

As shown in Figure 7, the fluorescence emission 55 advancing to the perforated mirror 44 is reflected downward by the perforated mirror 44 formed as a concave mirror and advances to the filter 62b of a filter unit 58.

Since the filter 62b has a property of cutting off light having a wavelength of 532 nm but transmitting light having a wavelength longer than 532 nm, light having the same wavelength of 532 nm as that of the stimulating ray is cut off by the filter 62b and only light in the wavelength of the fluorescence emission 55 released from Rhodamine passes through the filter 62b to be photoelectrically detected by the photomultiplier 60.

Analog data produced by photoelectrically detecting fluorescence emission 55 with the photomultiplier 60 are converted by the A/D converter 63 into digital data and the digital data are fed to a data processing apparatus 64.

When a predetermined time, for example, several microseconds, has passed after the second laser stimulating ray source 32 was turned on, the control unit 80 outputs a drive stop signal to the second laser stimulating ray source 32, thereby turning it off and outputs a drive signal to the main scanning stepping motor 75, thereby moving the optical head 45 by one pitch equal to the distance between neighboring absorptive regions 4 formed in the substrate 2 of the biochemical analysis unit 1.

When the control unit 80 determines based on a detection signal indicating the position of the optical head 45 input from the linear encoder 77 that the optical head 45 has been moved by one pitch equal to the distance between neighboring absorptive regions 4 formed in the substrate 2 of the biochemical analysis unit 1 and has reached a position where a laser beam 34 can be projected onto a second absorptive region 4 next to the first absorptive region 4 formed in the substrate 2 of the biochemical analysis unit 1, it outputs a drive signal to the second laser stimulating ray source 32 to turn it on, thereby causing the laser beam 34 to excite a fluorescent substance, for example, Rhodamine, contained in the second absorptive region 4 formed in the substrate 2 of the biochemical analysis unit 1 next to the first absorptive region 4.

Similarly to the above, the second absorptive region 4 formed in the substrate 2 of the biochemical analysis unit 1 is irradiated with the laser beam 34 for a predetermined time and when fluorescence emission 55 released from the second absorptive region 4 is photoelectrically detected by the photomultiplier 60 and analog data are produced, the control unit 80 outputs a drive stop signal to the second laser stimulating ray source 32, thereby turning it off and outputs a drive signal to the main scanning stepping motor 75, thereby moving the optical head 45 by one pitch equal to the distance between neighboring absorptive regions 4 formed in the substrate 2 of the biochemical analysis unit 1.

In this manner, the on and off operation of the second laser stimulating ray source 32 is repeated in synchronism with the intermittent movement of the optical head 45 and when the control unit 80 determines based on a detection signal indicating the position of the optical head 45 input from the linear encoder 77 that the optical head 45 has been moved by one scanning line in the main scanning direction and that the absorptive regions 4 included in a first line of the absorptive regions 4 formed in the substrate 2 of the biochemical analysis unit 1 have been scanned with the laser beam 34, it outputs a drive signal to the main scanning stepping motor 75, thereby returning the optical head 45 to its original position and outputs a drive signal to the sub-scanning pulse motor 71, thereby causing it to move the movable base plate 73 by one scanning line in the sub-scanning direction.

When the control unit 80 determines based on a detection signal indicating the position of the optical head 45 input from the linear encoder 77 that the optical head 45 has been returned to its original position and determines that the movable base plate 73 has been moved by one scanning line in the sub-scanning direction, similarly to the manner in which the absorptive regions 4 included in the first line of the absorptive regions 4 formed in the substrate 2 of the biochemical analysis unit 1 were sequentially irradiated with the laser beam 34 emitted from the second laser stimulating ray source 32, the absorptive regions 4 included in a second line of the absorptive regions 4 formed in the substrate 2 of the biochemical analysis unit 1 are sequentially irradiated with the laser beam 34 emitted from the second laser stimulating ray source 32, thereby exciting Rhodamine contained in the absorptive regions 4 included in the second line and fluorescence emission 55 released from the absorptive regions 4 included in the second line is sequentially and photoelectrically detected by the photomultiplier 60.

Analog data produced by photoelectrically detecting fluorescence emission 55 with the photomultiplier 60 are converted by the A/D converter 63 into digital data and the digital data are fed to the data processing apparatus 64.

When all of the absorptive regions 4 formed in the substrate 2 of the biochemical analysis unit 1 have been scanned with the laser beam 34 to excite Rhodamine contained in the absorptive regions 4 formed in the substrate 2 of the biochemical analysis unit 1 and digital data produced by photoelectrically detecting fluorescence emission 55 released from the absorptive regions 4 by the photomultiplier 60 to produce analog data and digitizing the analog data by the A/D converter 63 have been forwarded to the data processing apparatus 64, the control unit 80 outputs a drive stop signal to the second laser stimulating ray source 32, thereby turning it off.

As described above, fluorescence data recorded in a number of the absorptive regions 4 formed in the substrate 2 of the biochemical analysis unit 1 are read by the scanner and biochemical analysis data are produced.

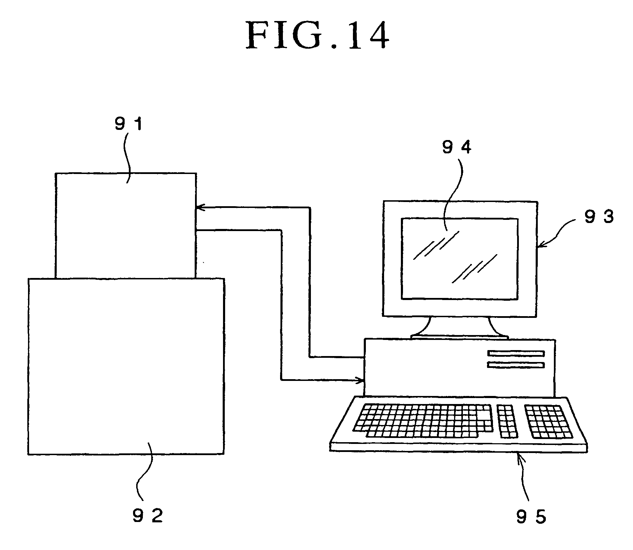

Figure 14 is a schematic front view showing a data producing system for reading chemiluminescence data recorded in a number of the absorptive regions 4 formed in the substrate 2 of the biochemical analysis unit 1, and producing biochemical analysis data.

The data producing system shown in Figure 14 is constituted to be able to not only read reading chemiluminescence data recorded in a number of the absorptive regions 4 formed in the biochemical analysis unit 1, thereby producing biochemical analysis data but also read fluorescence data of a fluorescent substance such as a fluorescent dye recorded in a number of the absorptive regions 4 formed in the biochemical analysis unit 1, thereby producing biochemical analysis data.

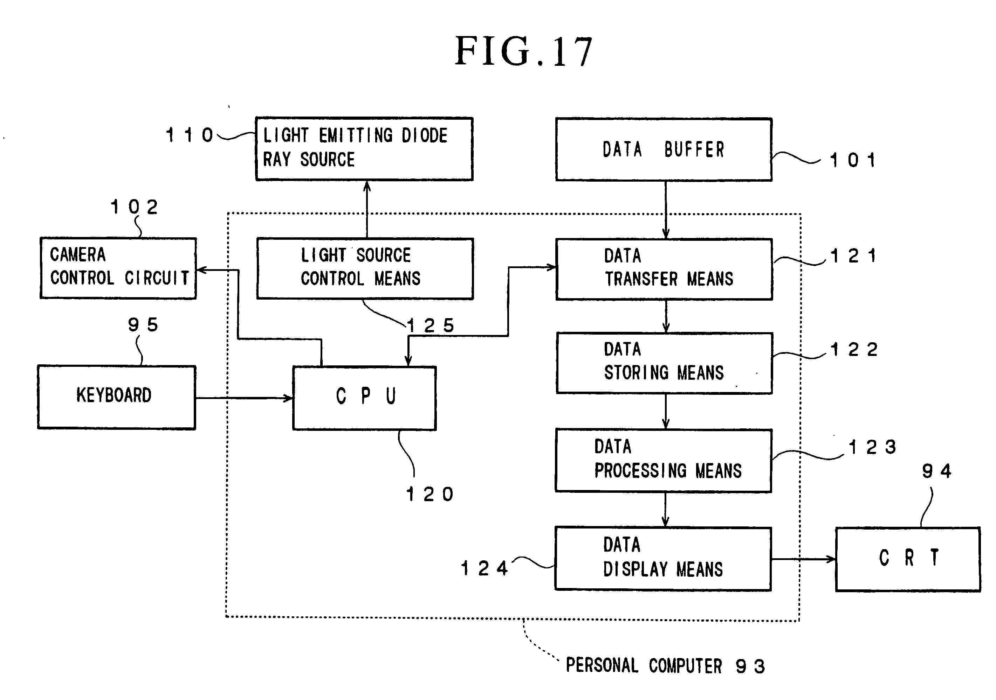

As shown in Figure 14, the data producing system includes a cooled CCD camera 91, a dark box 92 and a personal computer 93. As shown in Figure 17, the personal computer 93 is equipped with a CRT 94 and a keyboard 95.

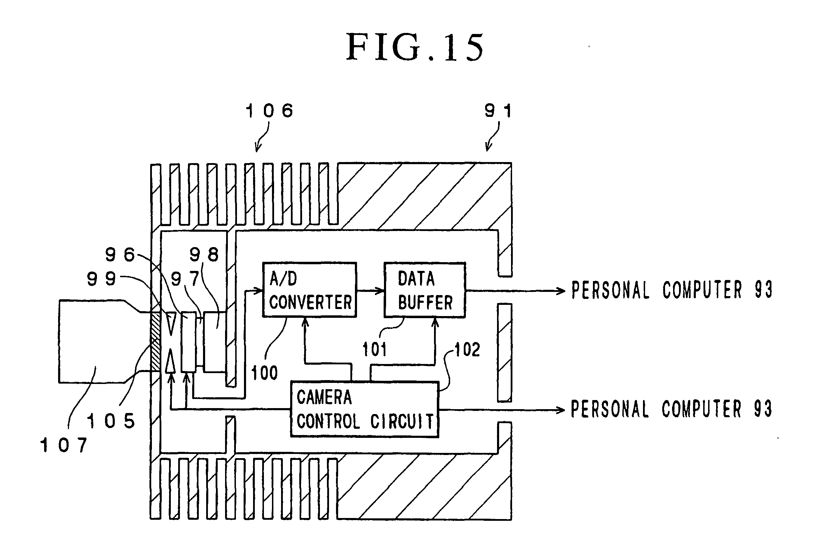

Figure 15 is a schematic longitudinal cross sectional view showing the cooled CCD camera 91 of the data producing system.

As shown in Figure 15, the cooled CCD camera 91 includes a CCD 96, a heat transfer plate 97 made of metal such as aluminum, a Peltier element 98 for cooling the CCD 96, a shutter 99 disposed in front of the CCD 96, an A/D converter 100 for converting analog data produced by the CCD 96 to digital data, a data buffer 101 for temporarily storing the data digitized by the A/D converter 100, and a camera control circuit 102 for controlling the operation of the cooled CCD camera 91.

An opening formed between the dark box 92 and the cooled CCD camera 91 is closed by a glass plate 105 and the periphery of the cooled CCD camera 91 is formed with heat dispersion fins 106 over substantially its entire length for dispersing heat.

A camera lens 107 disposed in the dark box 92 is mounted on the front surface of the glass plate 105 disposed in the cooled CCD camera 91.

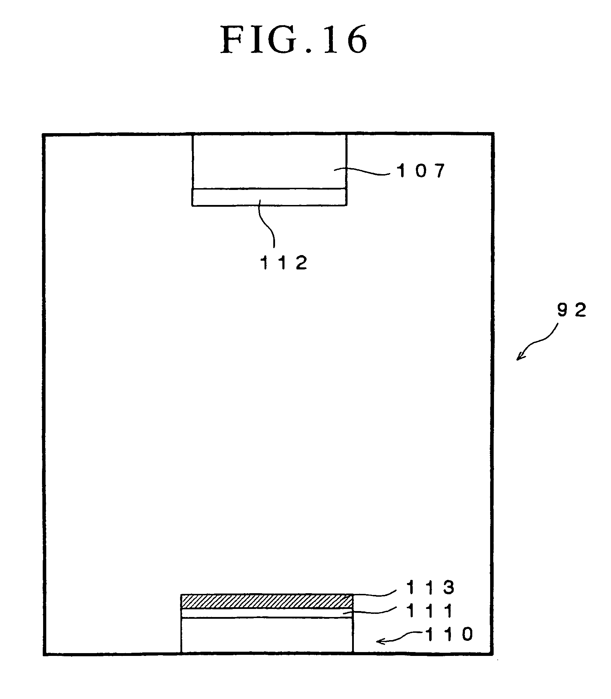

Figure 16 is a schematic vertical cross sectional view showing the dark box 92 of the data producing system.

As shown in Figure 16, the dark box 92 is equipped with a light emitting diode stimulating ray source 110 for emitting a stimulating ray. The light emitting diode stimulating ray source 110 is provided with a filter 111 detachably mounted thereon and a diffusion plate 113 mounted on the upper surface of the filter 111. The stimulating ray is emitted via the diffusion plate 113 toward a biochemical analysis unit (not shown) placed on the diffusion plate 113 so as to ensure that the biochemical analysis unit can be uniformly irradiated with the stimulating ray. The filter 111 has a property of cutting light components having a wavelength not close to that of the stimulating ray and harmful to the stimulation of a fluorescent substance and transmitting through only light components having a wavelength in the vicinity of that of the stimulating ray. A filter 112 for cutting light components having a wavelength in the vicinity of that of the stimulating ray is detachably provided on the front surface of the camera lens 107.

Figure 17 is a block diagram of the personal computer 93 of the data producing system and peripheral devices thereof.

As shown in Figure 17, the personal computer 93 includes a CPU 120 for controlling the exposure of the cooled CCD camera 91, a data transferring means 121 for reading the data produced by the cooled CCD camera 91 from the data buffer 101, a storing means 122 for storing data, a data processing means 123 for effecting data processing on the digital data stored in the data storing means 122, and a data displaying means 124 for displaying visual data on the screen of the CRT 94 based on the digital data stored in the data storing means 122.

The light emitting diode stimulating ray source 110 is controlled by a light source control means 125 and an instruction signal can be input via the CPU 120 to the light source control means 125 through the keyboard 85. The CPU 120 is constituted so as to output various signals to the camera controlling circuit 103 of the cooled CCD camera 91.

The data producing system shown in Figures 14 to 17 is constituted so as to detect chemiluminescence emission generated by the contact of an enzyme labeling an antibody to a hapten bound by an antigen-antibody reaction with the hapten such as digoxigenin labeling a substance derived from a living organism and selectively hybridized with specific binding substances contained in a number of the absorptive regions 4 of the biochemical analysis unit 1 and a chemiluminescent substrate, with the CCD 96 of the cooled CCD camera 91 through the camera lens 107, thereby reading chemiluminescence data recorded in a number of the absorptive regions 4 of the biochemical analysis unit 1 to produce biochemical analysis data, and irradiate the biochemical analysis unit 1 with a stimulating ray emitted from the light emitting diode stimulating ray source 110 and detect fluorescence emission released from a fluorescent substance such as a fluorescent dye contained in a number of the absorptive regions 4 formed in the substrate 2 of the biochemical analysis unit 1 upon being stimulated, with the CCD 96 of the cooled CCD camera 91 through a camera lens 107, thereby reading fluorescence data recorded in a number of the absorptive regions 4 of the biochemical analysis unit 1 to produce biochemical analysis data.

When biochemical analysis data are to be produced by reading chemiluminescence data, the filter 112 is removed and while the light emitting diode stimulating ray source 110 is kept off, the biochemical analysis unit 1 is placed on the diffusion plate 113. At this time, the biochemical analysis unit 1 is releasing chemiluminescence emission as a result of contact of a labeling enzyme labeling an antibody selectively contained in a number of the absorptive regions 4 formed in the substrate 2 of the biochemical analysis unit 1 and a chemiluminescent substrate.

The lens focus is then adjusted by the user using the camera lens 107 and the dark box 92 is closed.

When an exposure start signal is input by the user through the keyboard 95, the exposure start signal is input through the CPU 120 to the camera control circuit 102 of the cooled CCD camera 91 so that the shutter 99 is opened by the camera control circuit 102, whereby the exposure of the CCD 96 is started.

Chemiluminescence emission released from a number of the absorptive regions 4 formed in the substrate 2 of the biochemical analysis unit 1 impinges on the light receiving surface of the CCD 96 of the cooled CCD camera 91 via the camera lens 107, thereby forming an image on the light receiving surface.

The CCD 96 receives light of the thus formed image and accumulates it in the form of electric charges therein.

In this embodiment, since the substrate 2 of the biochemical analysis unit 1 is made of stainless steel and is capable of attenuating light energy, it is possible to reliably prevent chemiluminescence emission released from the labeling enzyme contained in each of the absorptive regions 4 from being scattered in the substrate 2 of the biochemical analysis unit 1 and mixed with chemiluminescence emission released from a labeling enzyme contained in the neighboring absorptive regions 4.

When a predetermined exposure time has passed, the CPU 120 outputs an exposure completion signal to the camera control circuit 102 of the cooled CCD camera 91.

When the camera controlling circuit 102 receives the exposure completion signal from the CPU 120, it transfers analog data accumulated in the CCD 96 in the form of electric charge to the A/D converter 100 to cause the A/D converter 100 to digitize the data and to temporarily store the thus digitized data in the data buffer 101.

At the same time, the CPU 120 outputs a data transfer signal to the data transferring means 121 to cause it to read out the digital data from the data buffer 101 of the cooled CCD camera 91 and to store them in the data storing means 122.

When the user inputs a data processing signal through the keyboard 95, the CPU 120 outputs the digital data stored in the data storing means 122 to the data processing means 123 and causes the data processing means 123 to effect data processing on the digital data in accordance with the user's instructions and store the thus processed digital data in the data storing means 122.

When the user then input a data display signal through the keyboard 81, the CPU 120 outputs a data display signal to the displaying means 124 and causes the displaying means 124 to display biochemical analysis data on the screen of the CRT 94 based on the digital data stored in the data storing means 122.

In this manner, chemiluminescent data recorded in a number of the absorptive regions 4 formed in the substrate 2 of the biochemical analysis unit 1 are read and biochemical analysis data are produced.

On the other hand, when biochemical analysis data are to be produced by reading fluorescence data recorded in a number of the absorptive regions 4 formed in the substrate 2 of the biochemical analysis unit 1, the biochemical analysis unit 1 is first placed on the diffusion plate 113.

The light emitting diode stimulating ray source 110 is then turned on by the user and the lens focus is adjusted using the camera lens 107. The dark box 92 is then closed.

When the user inputs an exposure start signal through the keyboard 95, the light emitting diode stimulating ray source 110 is again turned on by the light source control means 125, thereby emitting a stimulating ray toward the biochemical analysis unit 1.

At the same time, the exposure start signal is input via the CPU 120 to the camera control circuit 102 of the cooled CCD camera 91 and the shutter 99 is opened by the camera control circuit 102, whereby the exposure of the CCD 96 is started.

The stimulating ray emitted from the light emitting diode stimulating ray source 110 passes through the filter 111, whereby light components of wavelengths not in the vicinity of that of the stimulating ray are cut. The stimulating ray then passes through the diffusion plate 113 to be made uniform light and the biochemical analysis unit 1 is irradiated with the uniform stimulating ray.

When the biochemical analysis unit 1 is irradiated with the stimulating ray, a fluorescent substance such as a fluorescent dye selectively contained in a number of the absorptive regions 4 formed in the substrate 2 of the biochemical analysis unit 1 is stimulated by the stimulating ray, thereby releasing fluorescence emission from a number of the absorptive regions 4 of the biochemical analysis unit 1.

The fluorescence emission released from a number of the absorptive regions 4 formed in the substrate 2 of the biochemical analysis unit 1 impinges on the light receiving surface of the CCD 96 of the cooled CCD camera 91 through the filter 112 and the camera lens 107 and forms an image thereon.

Since light components of wavelength equal to the stimulating ray wavelength are cut by the filter 112, only fluorescence emission released from the fluorescent substance such as a fluorescent dye contained in a number of the absorptive regions 4 of the biochemical analysis unit 1 is received by the CCD 96.

The CCD 96 receives light of the thus formed image and accumulates it in the form of electric charges therein.

In this embodiment, since the substrate 2 of the biochemical analysis unit 1 is made of stainless steel and is capable of attenuating light energy, it is possible to reliably prevent fluorescence emission released from a fluorescent substance contained in each of the absorptive regions 4 from being scattered in the substrate 2 of the biochemical analysis unit 1 and mixed with fluorescence emission released from a fluorescent substance contained in the neighboring absorptive regions 4.

When a predetermined exposure time has passed, the CPU 120 outputs an exposure completion signal to the camera control circuit 102 of the cooled CCD camera 91.

When the camera controlling circuit 102 receives the exposure completion signal from the CPU 120, it transfers analog data accumulated in the CCD 96 in the form of electric charge to the A/D converter 100 to cause the A/D converter 100 to digitize the data and to temporarily store the thus digitized data in the data buffer 101.

At the same time, the CPU 120 outputs a data transfer signal to the data transferring means 121 to cause it to read out the digital data from the data buffer 101 of the cooled CCD camera 91 and to store them to the data storing means 122.

When the user inputs a data processing signal through the keyboard 95, the CPU 120 outputs the digital data stored in the data storing means 122 to the data processing means 123 and causes the data processing means 123 to effect data processing on the digital data in accordance with the user's instructions and store the thus processed digital data in the data storing means 122.

When the user then input a data display signal through the keyboard 81, the CPU 120 outputs a data display signal to the displaying means 124 and causes the displaying means 124 to display biochemical analysis data on the screen of the CRT 94 based on the digital data stored in the data storing means 122.

In this manner, fluorescent data recorded in a number of the absorptive regions 4 formed in the substrate 2 of the biochemical analysis unit 1 are read and biochemical analysis data are produced.

According to this embodiment, since the hybridization reaction solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, it is possible to markedly increase the moving rate of a substance derived from a living organism and contained in the hybridization reaction solution through the absorptive regions 4 of the biochemical analysis unit 1 in comparison with the case of moving a substance derived from a living organism and contained in the hybridization reaction solution only by convection or diffusion and hybridizing specific binding substances. Therefore, since it is possible to markedly increase the reaction rate of hybridization and it is possible to markedly increase the possibility of association of the substance derived from a living organism and contained in the hybridization reaction solution with the specific binding substances contained in deep portions of a number of the absorptive regions 4 of the biochemical analysis unit 1, the substance derived from a living organism and contained in the hybridization reaction solution can be hybridized with the specific binding substances fixed in a number of the absorptive regions 4 of the biochemical analysis unit 1 in a desired manner.

Further, according to this embodiment, since the antibody solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, it is possible to markedly increase the moving rate of an antibody contained in the antibody solution through the absorptive regions 4 of the biochemical analysis unit 1 in comparison with the case of moving an antibody contained in the antibody solution only by convection or diffusion and binding a hapten. Therefore, since it is possible to markedly increase the reaction rate of an antigen-antibody reaction and it is possible to markedly increase the possibility of association of an antibody to a hapten contained in the antibody solution with the hapten labeling a substance derived from a living organism and selectively hybridized with specific binding substances contained in deep portions of a number of the absorptive regions 4 of the biochemical analysis unit 1, the antibody to a hapten contained in the antibody solution can be bound by an antigen-antibody reaction in a desired manner with the labeling a substance derived from a living organism and selectively hybridized with specific binding substances contained in the absorptive regions 4 of the biochemical analysis unit 1.

Furthermore, according to this embodiment, since the cleaning solution contained in the space on one side of the biochemical analysis unit 1 held at the biochemical analysis unit holding section 7 of the reaction vessel 8 is pressurized and is forcibly moved so as to cut through a number of the absorptive regions 4 formed in the substrate 2 of the biochemical analysis unit 1 every time the piston 15 of the syringe 16 is moved vertically, even if a substance derived from a living organism which should not be hybridized with specific binding substances absorbed in the absorptive regions 4 formed in the substrate 2 of the biochemical analysis unit 1 has been absorbed in the absorptive regions 4 during the process of hybridization, it is possible to efficiently peel off and remove the substance derived from a living organism which should not be hybridized with specific binding substances absorbed in the absorptive regions 4 from a number of the absorptive regions 4 of the biochemical analysis unit 1. Further, even if an antibody which should not be bonded with the hapten labeling a substance derived from a living body and selectively hybridized with specific binding substances absorbed in the absorptive regions 4 of the biochemical analysis unit 1 has been absorbed in the absorptive regions 4 formed in the substrate 2 of the biochemical analysis unit 1 during the process of the antigen-antibody reaction, it is possible to efficiently peel off and remove the antibody which should not be bonded with the hapten from a number of the absorptive regions 4 of the biochemical analysis unit 1. Therefore, the efficiency of the cleaning operation can be markedly improved.

Moreover, according to this embodiment, it is possible to record chemiluminescent data in a number of the absorptive regions 4 of the biochemical analysis unit 1 using the same reactor by selectively hybridizing a substance derived from a living organism and labeled with a hapten such as digoxigenin with specific binding substances absorbed in a number of the absorptive regions 4 of the biochemical analysis unit 1 and further binding by an antigen-antibody reaction an antibody to the hapten such as digoxigenin labeled with an enzyme which generates chemiluminescence emission when it contacts a chemiluminescent substrate with the hapten such as digoxigenin labeling the substance derived with a living organism. Therefore, it is possible to very efficiently record chemiluminescent data in a number of the absorptive regions 4 of the biochemical analysis unit 1. Further, since a substance derived from a living organism is labeled with a hapten such as digoxigenin having a low molecular weight and the substance derived from a living organism is selectively hybridized with specific binding substances absorbed in a number of the absorptive regions 4 of the biochemical analysis unit 1, it is possible to hybridize specific binding substances and a substance derived from a living organism in a desired manner and, therefore, it is possible to produce biochemical analysis data having an excellent quantitative characteristic by reading chemiluminescent data.

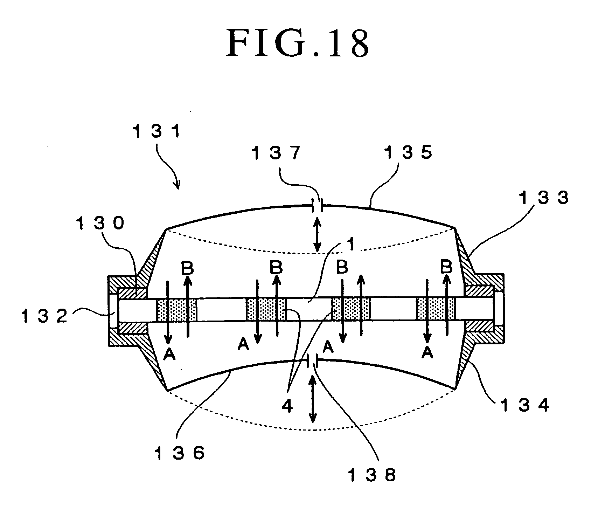

Figure 18 is a schematic longitudinal cross-sectional view showing a reactor used for conducting a receptor-ligand association reaction which is another preferred embodiment of the present invention.

As shown in Figure 18, the reactor according to this embodiment includes a reaction vessel 131 in which a biochemical analysis unit holding section 130 is provided for holding the biochemical analysis unit 1. The biochemical analysis unit holding section 130 is constituted so as to be able to prevent leakage of a liquid.

The reaction vessel 131 includes a main body 132, an upper half portion 133 and a lower half portion 134 The upper half portion 133 is detachably mounted on the main body 132 of a reaction vessel 131.

As shown in Figure 18, the upper half portion 133 is provided with a diaphragm 135 formed of a metal plate having flexibility and the lower half portion 134 is provided with a diaphragm 136 formed of a metal plate having flexibility.

The diaphragm 135 and the diaphragm 136 can be deformed by an air cylinder (not shown) and can assume positions indicated by a solid line and positions indicated by the broken line in Figure 18.

A substantially center portion of the diaphragm 135 of the upper half portion 133 is formed with an air vent opening 137 which can be opened and closed by a valve (not shown) and a substantially center portion of the diaphragm 136 of the lower half portion 134 is formed with a solution feeding and discharging opening 138 which can be opened and closed by a valve (not shown).

In the thus constituted reactor according to this embodiment, a substance derived from a living body, labeled with a labeling substance and contained in a hybridization reaction solution selectively hybridizes specific binding substances contained in a number of the absorptive regions 4 of the biochemical analysis unit 1 in the following manner.

Similarly to the previous embodiment, in this embodiment, a hybridization reaction solution containing a substance derived from a living organism and labeled with a radioactive labeling substance, a substance derived from a living organism and labeled with a fluorescent substance such as a fluorescent dye and a substance derived from a living organism and labeled with a hapten such as digoxigenin is prepared.

When hybridization is to be performed, the upper half portion 133 of the reaction vessel 131 is removed from the main body 132 of the reaction vessel 131 and the biochemical analysis unit 1 including a number of the absorptive regions 4 in which specific binding substances are absorbed is attached to the biochemical analysis unit holding section 130 and held thereby.

When the biochemical analysis unit 1 has been set in the biochemical analysis unit holding section 130, the upper half portion 133 of the reaction vessel 131 is attached to the main body 132 of the reaction vessel 131.

With the diaphragm 135 and the diaphragm 136 held at the positions indicated by the solid line in Figure 18, the air vent opening 137 formed in the diaphragm 135 of the upper half portion 133 is opened to the atmosphere by opening the valve thereof (not shown), and the valve (not shown) of the solution feeding and discharging opening 138 formed in the diaphragm 136 of the lower half portion 134 is opened to feed a hybridizing reaction solution into the reaction vessel 131.

When the inside of the reaction vessel 131 has been completely filled with the hybridizing reaction solution and air has been completely discharged from the reaction vessel 131 through the air vent opening 137, the air vent opening 137 is closed by closing its valve (not shown), and the solution feeding and discharging opening 138 is closed by operating its valve (not shown).

The air cylinder (not shown) is then driven so that the diaphragm 135 and the diaphragm 136 are deformed and moved to the positions indicated by the broken line in Figure 18.

As a result, the hybridization reaction solution contained in the space above the biochemical analysis unit 1 held at the biochemical analysis unit holding section 130 of the reaction vessel 131 is pressurized and forcibly moved from the upper side of the biochemical analysis unit 1 held by the biochemical analysis unit holding section 130 toward the lower side thereof as indicated by arrows A in Figure 18 so as to cut through a number of the absorptive regions 4 formed in the substrate 2 of the biochemical analysis unit 1.

The air cylinder (not shown) is then driven so that the diaphragm 135 and the diaphragm 136 are deformed and moved to the positions indicated by the solid line in Figure 18.

As a result, the hybridization reaction solution contained in the space below the biochemical analysis unit 1 held at the biochemical analysis unit holding section 130 of the reaction vessel 131 is pressurized and forcibly moved from the lower side of the biochemical analysis unit 1 held by the biochemical analysis unit holding section 130 toward the upper side thereof as indicated by arrows B in Figure 18 so as to cut through a number of the absorptive regions 4 formed in the substrate 2 of the biochemical analysis unit 1.

When similar operations have been repeated a predetermined number of times, hybridization is completed.

In this manner, since the hybridization reaction solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, it is possible to markedly increase the moving rate of a substance derived from a living organism and contained in the hybridization reaction solution through the absorptive regions 4 of the biochemical analysis unit 1 in comparison with the case of moving a substance derived from a living organism and contained in the hybridization reaction solution only by convection or diffusion and hybridizing specific binding substances. Therefore, since it is possible to markedly increase the reaction rate of hybridization and it is possible to markedly increase the possibility of association of the substance derived from a living organism and contained in the hybridization reaction solution with the specific binding substances contained in deep portions of a number of the absorptive regions 4 of the biochemical analysis unit 1, the substance derived from a living organism and contained in the hybridization reaction solution can be hybridized with the specific binding substances fixed in a number of the absorptive regions 4 of the biochemical analysis unit 1 in a desired manner.

When hybridization has been completed in this manner, then, while the diaphragm 135 and the diaphragm 136 are located at the positions indicated by the solid line in Figure 18, the air vent opening 137 formed in the diaphragm 135 of the upper half portion 133 is opened to the atmosphere by opening its valve (not shown), and the valve (not shown) of the solution feeding and discharging opening 138 formed in the diaphragm 136 of the lower half portion 134 is opened so that the hybridizing reaction solution is discharged from the reaction vessel 131.

When the hybridizing reaction solution has been discharged from the reaction vessel 131, a cleaning solution is fed into the reactor through the solution feeding and discharging opening 138 formed in the diaphragm 136 of the lower half portion 134.

When the inside of the reaction vessel 131 has been completely filled with the cleaning solution and air has been completely discharged from the reaction vessel 131 through the air vent opening 137, the air vent opening 137 is closed by operating its valve (not shown), and the solution feeding and discharging opening 138 is closed by operating its valve.

The air cylinder (not shown) is then driven so that the diaphragm 134 and the diaphragm 135 are deformed and moved to the positions indicated by the broken line in Figure 18.

As a result, the cleaning solution contained in the space above the biochemical analysis unit 1 held at the biochemical analysis unit holding section 130 of the reaction vessel 131 is pressurized and forcibly moved from the upper side of the biochemical analysis unit 1 held by the biochemical analysis unit holding section 130 toward the lower side thereof as indicated by the arrows A in Figure 18 so as to cut through a number of the absorptive regions 4 formed in the substrate 2 of the biochemical analysis unit 1.

The air cylinder (not shown) is then driven so that the diaphragm 134 and the diaphragm 135 are deformed and moved to the positions indicated by the solid line in Figure 18.

As a result, the cleaning solution contained in the space below the biochemical analysis unit 1 held at the biochemical analysis unit holding section 130 of the reaction vessel 131 is pressurized and forcibly moved from the lower side of the biochemical analysis unit 1 held by the biochemical analysis unit holding section 130 toward the upper side thereof as indicated by the arrows B in Figure 18 so as to cut through a number of the absorptive regions 4 formed in the substrate 2 of the biochemical analysis unit 1.

When similar operations have been repeated a predetermined number of times, the cleaning operation is completed.

In this manner, since the cleaning solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, even if a substance derived from a living organism which should not be hybridized with specific binding substances absorbed in the absorptive regions 4 formed in the substrate 2 of the biochemical analysis unit 1 has been absorbed in the absorptive regions 4 during the process of hybridization, it is possible to efficiently peel off and remove the substance derived from a living organism which should not be hybridized with specific binding substances absorbed in the absorptive regions 4 from a number of the absorptive regions 4 of the biochemical analysis unit 1 and markedly improve the efficiency of the cleaning operation.

When the cleaning operation has been completed in this manner, then, while the diaphragm 135 and the diaphragm 136 are located at the positions indicated by the solid line in Figure 18, the air vent opening 137 formed in the diaphragm 135 of the upper half portion 133 is opened to the atmosphere by opening its valve (not shown), and the valve (not shown) of the solution feeding and discharging opening 138 formed in the diaphragm 136 of the lower half portion 134 is opened so that the cleaning solution is discharged from the reaction vessel 131.

As described above, radiation data of a radioactive labeling substance and a fluorescence data of a fluorescent substance such as a fluorescent dye are recorded in a number of the absorptive regions 4 formed in the substrate 2 of the biochemical analysis unit 1.

Similarly to the previous embodiment, the fluorescence data recorded in a number of the absorptive regions 4 of the biochemical analysis unit 1 are read by the scanner shown in Figures 6 to 13 and biochemical analysis data are produced.

On the other hand, similarly to the previous embodiment, the radiation data recorded in a number of the absorptive regions 4 of the biochemical analysis unit 1 are transferred onto the stimulable phosphor sheet 20 shown in Figure 4 and read by the scanner shown in Figures 6 to 13, thereby producing biochemical analysis data.

To the contrary, in order to record chemiluminescence data in a number of the absorptive regions 4 formed in the substrate 2 of the biochemical analysis unit 1, an antibody solution containing an antibody to the hapten labeled with an enzyme which generates chemiluminescence emission when it contacts a chemiluminescent substrate is further prepared and fed into the reaction vessel 131 and the antibody to the hapten labeled with an enzyme which generates chemiluminescence emission when it contacts a chemiluminescent substrate is bound with the hapten such as digoxigenin labeling a substance derived from a living organism selectively hybridized with specific binding substances absorbed in a number of the absorptive regions 4 formed in the substrate 2 of the biochemical analysis unit 1 by the an antigen-antibody reaction.

Specifically, when the discharge of the cleaning solution from the reaction vessel 131 has been completed, an antibody solution containing an antibody to the hapten labeled with an enzyme which generates chemiluminescence emission when it contacts a chemiluminescent substrate is fed into the reaction vessel 131 through the solution feeding opening 138 formed in the diaphragm 136 of the lower half portion 134.

When the inside of the reaction vessel 131 has been filled with the antibody solution containing an antibody to the hapten labeled with an enzyme which generates chemiluminescence emission when it contacts a chemiluminescent substrate and air has been completely discharged from the reaction vessel 131 through the air vent opening 137, the air vent opening 137 is closed by operating its valve (not shown), and the solution feeding and discharging opening 138 is closed by operating its valve (not shown).

The air cylinder (not shown) is then driven so that the diaphragm 134 and the diaphragm 135 are deformed and moved to the positions indicated by the broken line in Figure 18.

As a result, the antibody solution contained in the space above the biochemical analysis unit 1 held at the biochemical analysis unit holding section 130 of the reaction vessel 131 is pressurized and forcibly moved from the upper side of the biochemical analysis unit 1 held by the biochemical analysis unit holding section 130 toward the lower side thereof as indicated by the arrows A in Figure 18 so as to cut through a number of the absorptive regions 4 formed in the substrate 2 of the biochemical analysis unit 1.

The air cylinder (not shown) is then driven so that the diaphragm 134 and the diaphragm 135 are deformed and moved to the positions indicated by the solid line in Figure 18.

As a result, the antibody solution contained in the space below the biochemical analysis unit 1 held at the biochemical analysis unit holding section 130 of the reaction vessel 131 is pressurized and forcibly moved from the lower side of the biochemical analysis unit 1 held by the biochemical analysis unit holding section 130 toward the upper side thereof as indicated by the arrows B in Figure 18 so as to cut through a number of the absorptive regions 4 formed in the substrate 2 of the biochemical analysis unit 1.

Similar operations are repeated a predetermined number of times, whereby an antibody to a hapten labeled with an enzyme which generates chemiluminescence emission when it contacts a chemiluminescent substrate is bound by an antigen-antibody reaction with the hapten such as digoxigenin labeling a substance derived from a living organism selectively hybridized with specific binding substances absorbed in a number of the absorptive regions 4 formed in the substrate 2 of the biochemical analysis unit 1 and the antigen-antibody reaction is completed.

In this manner, since the antibody solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, it is possible to markedly increase the moving rate of an antibody contained in the antibody solution through the absorptive regions 4 of the biochemical analysis unit 1 in comparison with the case of moving an antibody contained in the antibody solution only by convection or diffusion and binding a hapten. Therefore, since it is possible to markedly increase the reaction rate of an antigen-antibody reaction and it is possible to markedly increase the possibility of association of an antibody to a hapten contained in the antibody solution with the hapten labeling a substance derived from a living organism selectively hybridized with specific binding substances absorbed in a number of the absorptive regions 4 formed in the substrate 2 of the biochemical analysis unit 1, it is possible to bind in a desired manner by an antigen-antibody reaction an antibody to a hapten contained in the antibody solution with the hapten labeling a substance derived from a living organism selectively hybridized with specific binding substances absorbed in a number of the absorptive regions 4 formed in the substrate 2 of the biochemical analysis unit 1.

When the antigen-antibody reaction has been completed in this manner, then, while the diaphragm 135 and the diaphragm 136 are located at the positions indicated by the solid line in Figure 18, the air vent opening 137 formed in the diaphragm 135 of the upper half portion 133 is opened to the atmosphere by opening its valve (not shown), and the valve (not shown) of the solution feeding and discharging opening 138 formed in the diaphragm 136 of the lower half portion 134 is opened so that the antibody solution is discharged from the reaction vessel 131.

When the antibody solution has been discharged from the reaction vessel 131, a cleaning solution is fed into the reaction vessel 131 through the solution feeding and discharging opening 138 formed in the diaphragm 136 of the lower half portion 134.

When the inside of the reaction vessel 131 has been completely filled with the cleaning solution and air has been completely discharged from the reaction vessel 131 through the air vent opening 137, the air vent opening 137 is closed by operating its valve (not shown), and the solution feeding and discharging opening 138 is closed by operating its valve (not shown). Then, the diaphragm 135 and the diaphragm 136 are alternately deformed between the position indicated by a solid line and the position indicated by the broken line in Figure 18, whereby a number of the absorptive regions 4 formed in the substrate 2 of the biochemical analysis unit 1 are cleaned with the cleaning solution similarly to the above.

Therefore, since the cleaning solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, even if an antibody which should not be bonded with the hapten labeling a substance derived from a living body and selectively hybridized with specific binding substances absorbed in the absorptive regions 4 of the biochemical analysis unit 1 has been absorbed in the absorptive regions 4 formed in the substrate 2 of the biochemical analysis unit 1 during the process of the antigen-antibody reaction, it is possible to efficiently peel off and remove the antibody which should not be bonded with the hapten from a number of the absorptive regions 4 of the biochemical analysis unit 1 and the efficiency of the cleaning operation can be markedly improved.

When the cleaning operation has been completed in this manner, then, while the diaphragm 135 and the diaphragm 136 are located at the positions indicated by the solid line in Figure 18, the air vent opening 137 formed in the diaphragm 135 of the upper half portion 133 is opened to the atmosphere by operating its valve (not shown) and the valve (not shown) of the solution feeding and discharging opening 138 formed in the diaphragm 136 of the lower half portion 134 is opened so that the cleaning solution is discharged from the reaction vessel 131.

As described above, chemiluminescence data are recorded in a number of the absorptive regions 4 formed in the substrate 2 of the biochemical analysis unit 1.

Similarly to the previous embodiment, chemiluminescence data recorded in a number of the absorptive regions 4 formed in the substrate 2 of the biochemical analysis unit 1 are read by the cooled CCD camera 91 of the data producing system shown in Figures 14 to 17 and biochemical analysis data are produced.

When the discharge of the cleaning solution from the reaction vessel 131 has been completed, the upper half portion 133 of the reaction vessel 131 is removed from the main body 132 of the reaction vessel 131 and the biochemical analysis unit 1 held at the biochemical analysis unit holding section 130 is lifted from the reaction vessel 131.

According to this embodiment, since the hybridization reaction solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, it is possible to markedly increase the moving rate of a substance derived from a living organism and contained in the hybridization reaction solution through the absorptive regions 4 of the biochemical analysis unit 1 in comparison with the case of moving a substance derived from a living organism and contained in the hybridization reaction solution only by convection or diffusion and hybridizing specific binding substances. Therefore, since it is possible to markedly increase the reaction rate of hybridization and it is possible to markedly increase the possibility of association of the substance derived from a living organism and contained in the hybridization reaction solution with the specific binding substances contained in deep portions of a number of the absorptive regions 4 of the biochemical analysis unit 1, the substance derived from a living organism and contained in the hybridization reaction solution can be hybridized with the specific binding substances fixed in a number of the absorptive regions 4 of the biochemical analysis unit 1 in a desired manner.

Further, according to this embodiment, since the antibody solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, it is possible to markedly increase the moving rate of an antibody contained in the antibody solution through the absorptive regions 4 of the biochemical analysis unit 1 in comparison with the case of moving an antibody contained in the antibody solution only by convection or diffusion and binding a hapten. Therefore, since it is possible to markedly increase the reaction rate of an antigen-antibody reaction and it is possible to markedly increase the possibility of association of an antibody to a hapten contained in the antibody solution with the hapten labeling a substance derived from a living organism selectively hybridized with specific binding substances absorbed in a number of the absorptive regions 4 formed in the substrate 2 of the biochemical analysis unit 1, it is possible to bind in a desired manner by an antigen-antibody reaction an antibody to a hapten contained in the antibody solution with the hapten labeling a substance derived from a living organism selectively hybridized with specific binding substances absorbed in a number of the absorptive regions 4 formed in the substrate 2 of the biochemical analysis unit 1.

Furthermore, according to this embodiment, since the cleaning solution contained in the space on one side of the biochemical analysis unit 1 held by the biochemical analysis unit holding section 130 of the reaction vessel 131 is pressurized and forcibly moved so as to cut through a number of the absorptive regions 4 formed in the substrate 2 of the biochemical analysis unit 1 every time the air cylinder (not shown) is driven so that the diaphragm 135 and the diaphragm 136 are deformed between the position indicated by a solid line and the position indicated by the broken line in Figure 18, even if a substance derived from a living organism which should not be hybridized with specific binding substances absorbed in the absorptive regions 4 formed in the substrate 2 of the biochemical analysis unit 1 has been absorbed in the absorptive regions 4 during the process of hybridization, it is possible to efficiently peel off and remove the substance derived from a living organism which should not be hybridized with specific binding substances absorbed in the absorptive regions 4 from a number of the absorptive regions 4 of the biochemical analysis unit 1. Further, even if an antibody which should not be bonded with the hapten labeling a substance derived from a living body and selectively hybridized with specific binding substances absorbed in the absorptive regions 4 of the biochemical analysis unit 1 has been absorbed in the absorptive regions 4 formed in the substrate 2 of the biochemical analysis unit 1 during the process of the antigen-antibody reaction, it is possible to efficiently peel off and remove the antibody which should not be bonded with the hapten from a number of the absorptive regions 4 of the biochemical analysis unit 1. Therefore, the efficiency of the cleaning operation can be markedly improved.

Moreover, according to this embodiment, it is possible to record chemiluminescent data in a number of the absorptive regions 4 of the biochemical analysis unit 1 using the same reactor by selectively hybridizing a substance derived from a living organism and labeled with a hapten such as digoxigenin with specific binding substances absorbed in a number of the absorptive regions 4 of the biochemical analysis unit 1 and further binding by an antigen-antibody reaction an antibody to the hapten such as digoxigenin labeled with an enzyme which generates chemiluminescence emission when it contacts a chemiluminescent substrate with the hapten such as digoxigenin labeling the substance derived with a living organism. Therefore, it is possible to very efficiently record chemiluminescent data in a number of the absorptive regions 4 of the biochemical analysis unit 1. Further, since a substance derived from a living organism is labeled with a hapten such as digoxigenin having a low molecular weight and the substance derived from a living organism is selectively hybridized with specific binding substances absorbed in a number of the absorptive regions 4 of the biochemical analysis unit 1, it is possible to hybridize specific binding substances and a substance derived from a living organism in a desired manner and, therefore, it is possible to produce biochemical analysis data having an excellent quantitative characteristic by reading chemiluminescent data.

### WORKING EXAMPLES AND COMPARATIVE EXAMPLES

Hereinafter, working examples and comparative examples will be set out in order to further clarify the advantages of the present invention.

### COMPARATIVE EXAMPLE 1

A solution of "pBR328-DNA" manufactured by Roche Ltd. having a concentration of 25 ng per microliter was spotted at 10 nanoliter per spot onto an absorptive substrate made of nylon 6, 6 to form absorptive regions spaced apart from each other by 1 mm at a density of 36 per 81 mm², thereby producing a biochemical analysis unit and the thus obtained biochemical analysis unit was accommodated in a box-like reaction vessel.

On the other hand, a hybridization buffer solution was prepared so as to contain 52 milliliter of sterilized aqua pura, 30 milliliter of a "20xSSC" manufactured by Nippon Gene Co. Ltd., 2 milliliter of EDTA (pH 8.0) having a concentration of 0.5 M, 10 milliliter of "50xdenhard's" solution, 5 milliliter of SDS solution having a concentration of 10 % and 1 milliliter of denatured DNA of salmon spermatozoa per 100 milliliter thereof.

Further, a solution of "pBR328-DNA" manufactured by Roche Ltd. having a concentration of 5 ng / microliter and labeled with digoxigenin was diluted by a TE buffer solution (a mixed solution of Tris-HCl having a concentration of 10 millimole and an EDTA solution having a concentration of 1 millimole) manufactured by Nippon Gene Co. Ltd., thereby preparing a solution of "pBR328-DNA" having a concentration of 5 pg / microliter and labeled with digoxigenin. Then, the hybridization buffer solution and the solution of "pBR328-DNA" having a concentration of 5 pg / microliter and labeled with digoxigenin were mixed at a ratio of 1000 to 1 by volume, thereby preparing a hybridization reaction solution.

The reaction vessel accommodating the biochemical analysis unit was filled with the thus prepared hybridization reaction solution and a hybridization reaction was preformed for three hours while the reaction vessel was vibrated in a cycle of ten times per minute.

After the hybridization reaction was completed, a cleaning solution was fed into the reaction vessel, thereby cleaning the biochemical analysis unit with the cleaning solution.

A blocking buffer solution manufactured by Roche Ltd. was diluted so that the concentration thereof became 1/10 with maleic acid buffer (Roche Ltd.) diluted with sterilized aqua pura so that the concentration thereof became 1/10 and an anti-digoxigenin-AP-conjugate solution manufactured by Roche Ltd. was diluted with the thus prepared blocking buffer solution so that the concentration thereof became 1/10,000, thereby preparing an antibody solution.

In order to prevent alkaline phosphatase bound with an antibody from binding portions of the absorptive regions other than portions with which "pBR328-DNA" labeled with digoxigenin were bound, the reaction vessel was first filled with the blocking buffer solution prepared in the above described manner and was vibrated in a cycle of ten times per minute for one hour.

The reaction vessel in which the biochemical analysis unit was accommodated was then filled with the antibody solution prepared in the above described manner and an antigen-antibody reaction was performed for one hour while the reaction vessel was vibrated in a cycle of ten times per minute, thereby binding alkaline phosphatase with "pBR328-DNA" labeled with digoxigenin.

After the antigen-antibody reaction was completed, a cleaning solution was fed into the reaction vessel, thereby cleaning the biochemical analysis unit with the cleaning solution. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

### COMPARATIVE EXAMPLE 2

The hybridization reaction and the antigen-antibody reaction were performed similarly to COMPARATIVE EXAMPLE 1 except that the hybridization reaction was performed for eighteen hours. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

### WORKING EXAMPLE 1

Absorptive regions were formed on the absorptive substrate similarly to COMPARATIVE EXAMPLE 1 and the thus obtained biochemical analysis unit was set in the reaction vessel of the reactor shown in Figure 3.

A hybridization reaction solution was prepared similarly to COMPARATIVE EXAMPLE 1 and the thus prepared hybridization reaction solution was fed into the reaction vessel.

After the reaction vessel was filled with the hybridization reaction solution, a hybridization reaction was performed by reciprocating the piston of the syringe.

The hybridization reaction was performed for three hours with the reciprocation rate of the piston set so that the hybridization reaction solution passed through the absorptive regions of the biochemical analysis unit at a rate of 0.25 cm/sec, 0.5 cm/sec or 1.0 cm/sec.

After the hybridization reaction was completed, a cleaning solution was fed into the reaction vessel, thereby cleaning the biochemical analysis unit with the cleaning solution. An antibody solution prepared similarly to COMPARATIVE EXAMPLE 1 was then fed into the reaction vessel and the antigen-antibody reaction was performed for one hour.

After the antigen-antibody reaction was completed, a cleaning solution was fed into the reaction vessel, thereby cleaning the biochemical analysis unit with the cleaning solution. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

### WORKING EXAMPLE 2

The hybridization reaction and the antigen-antibody reaction were performed similarly to WORKING EXAMPLE 1 except that the hybridization reaction was performed for eighteen hours. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

### WORKING EXAMPLE 3

Absorptive regions were formed by charging nylon 6, 6 in through-holes having a size of 0.0007 cm² and formed in a substrate made of SUS 304 at a density of 400 per 81 mm² and 10 nanoliter of a solution of "pBR328-DNA" manufactured by Roche Ltd. having a concentration of 25 ng per microliter was spotted into each of the absorptive regions, thereby producing a biochemical analysis unit. The thus obtained biochemical analysis unit was set in the reaction vessel of the reactor shown in Figure 3.

A hybridization reaction solution was prepared similarly to COMPARATIVE EXAMPLE 1 and the thus prepared hybridization reaction solution was fed into the reaction vessel.

After the reaction vessel was filled with the hybridization reaction solution, a hybridization reaction was performed by reciprocating the piston of the syringe.

The hybridization reaction was performed for three hours by setting the reciprocation rate of the piston so that the hybridization reaction solution passed through the absorptive regions of the biochemical analysis unit at a rate of 0.17 cm/sec.

After the hybridization reaction was completed, a cleaning solution was fed into the reaction vessel, thereby cleaning the biochemical analysis unit with the cleaning solution. An antibody solution prepared similarly to COMPARATIVE EXAMPLE 1 was then fed into he reaction vessel and the antigen-antibody reaction was performed for one hour.

After the antigen-antibody reaction was completed, a cleaning solution was fed into the reaction vessel, thereby cleaning the biochemical analysis unit with the cleaning solution. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

### WORKING EXAMPLE 4

The hybridization reaction and the antigen-antibody reaction were performed similarly to WORKING EXAMPLE 3 except that the hybridization reaction was performed for eighteen hours. The biochemical analysis unit lifted from the reaction vessel was then brought into contact with a chemiluminescent substrate and chemiluminescence emission released from the absorptive regions of the biochemical analysis unit was photoelectrically detected by the cooled CCD camera of the data producing system shown in Figures 14 to 17, thereby producing digital signals.

However, the hybridization reaction was performed by setting the reciprocation rate of the piston so that the hybridization reaction solution passed through the absorptive regions of the biochemical analysis unit at a rate of 0.17 cm/sec or 0.5 cm/sec.

The results of comparing the signal intensities of the digital signals produced in COMPARATIVE EXAMPLE 1, WORKING EXAMPLE 1 and WORKING EXAMPLE 2 are shown in Table 1.

Table 1 shows the signal intensities of the digital signals in WORKING EXAMPLE 1 and WORKING EXAMPLE 2 normalized by the signal intensities of the digital signals in COMPARATIVE EXAMPLE 1.

**Table 1**

| passing rate | 0.17cm/sec | 0.25cm/sec | 0.5cm/sec | 1.0cm/sec |
|---|---|---|---|---|
| WORKING EXAMPLE 1 | ― | 2.9 | 5.8 | 7.4 |
| WORKING EXAMPLE 3 | 1.6 | ― | ― | ― |

The results of comparing the signal intensities of the digital signals produced in COMPARATIVE EXAMPLE 2, WORKING EXAMPLE 3 and WORKING EXAMPLE 4 are shown in Table 2.

Table 2 shows the signal intensities of the digital signals in WORKING EXAMPLE 3 and WORKING EXAMPLE 4 normalized by the signal intensities of the digital signals in COMPARATIVE EXAMPLE 2.

**Table 2**

| passing rate | 0.17cm/sec | 0.25cm/sec | 0.5cm/sec | 1.0cm/sec |
|---|---|---|---|---|
| WORKING EXAMPLE 2 | ― | 2.0 | 3.5 | 4.6 |
| WORKING EXAMPLE 4 | 11 | ― | 15 | ― |

As shown in Table 1, it was found that the signal intensities of the digital signals produced in WORKING EXAMPLE 1 and WORKING EXAMPLE 2 in which the hybridization reaction was performed for three hours by a method according to the present invention was markedly increased in comparison with those produced in COMPARATIVE EXAMPLE 1 and that the efficiency of the hybridization reaction was markedly increased.

As shown in Table 2, it was found that the signal intensities of the digital signals produced in WORKING EXAMPLE 3 and the WORKING EXAMPLE 4 in which the hybridization reaction was performed for eighteen hours by a method according to the present invention was markedly increased in comparison with those produced in COMPARATIVE EXAMPLE 2 and that the efficiency of the hybridization reaction was markedly increased.

In particular, it was found that the signal intensities of the digital signals were higher as the rate of the hybridization reaction solution passing through the absorptive regions of the biochemical analysis unit was greater.

It was further found that the improvement in the signal intensities of the digital signals when the hybridization reaction was performed for three hours was significant and that the reaction rate of the hybridization reaction was markedly increased.

The present invention has thus been shown and described with reference to specific embodiments and examples. However, it should be noted that the present invention is in no way limited to the details of the described arrangements but changes and modifications may be made without departing from the scope of the appended claims.

For example, in the above described embodiments, radiation data, fluorescence data and chemiluminescence data are selectively recorded in a number of the absorptive regions 4 of the biochemical analysis unit 1 by selectively hybridizing a substance derived from a living organism and labeled with a radioactive labeling substance and a fluorescent substance with specific labeling substances absorbed in a number of the absorptive regions 4 of the biochemical analysis unit 1, selectively hybridizing a substance derived from a living organism and labeled with a hapten such as digoxigenin with specific labeling substances absorbed in a number of the absorptive regions 4 of the biochemical analysis unit 1 and further binding an antibody for the hapten labeled with an enzyme which generates chemiluminescence emission when it contacts a chemiluminescent substrate with the hapten such as digoxigenin labeling a substance derived from a living organism selectively hybridized with the specific binding substances fixed in a number of the absorptive regions 4 of the biochemical analysis unit 1 by an antigen-antibody reaction. However, the application of the present invention is not limited to such a hybridization reaction and an antigen-antibody reaction and the present invention can be applied to various kinds of hybridization reactions and various kinds of antigen-antibody reactions. Moreover, the present invention can be widely applied to various kinds of receptor-ligand association reactions.

Further, in the above described embodiments, chemiluminescence data are selectively recorded in a number of the absorptive regions 4 of the biochemical analysis unit 1 by selectively hybridizing a substance derived from a living organism and labeled with a hapten such as digoxigenin with specific labeling substances fixed in a number of the absorptive regions 4 of the biochemical analysis unit 1 and further binding an antibody to the hapten labeled with an enzyme which generates chemiluminescence emission when it contacts a chemiluminescent substrate with the hapten labeling a substance derived from a living organism and selectively hybridized with the specific binding substances fixed in number of the absorptive regions 4 of the biochemical analysis unit 1 by an antigen-antibody reaction. However, chemiluminescence data may be selectively recorded in a number of the absorptive regions 4 of the biochemical analysis unit 1 by selectively hybridizing a substance derived from a living body and labeled with a labeling substance which generates chemiluminescence emission when it contacts a chemiluminescent substrate with specific binding substances fixed in a number of the absorptive regions 4 of the biochemical analysis unit 1.

Furthermore, in the above described embodiments, fluorescence data are selectively recorded in a number of the absorptive regions 4 of the biochemical analysis unit 1 by selectively hybridizing a substance derived from a living organism and labeled with a fluorescent substance with specific labeling substances such as cDNAs fixed in a number of the absorptive regions 4 of the biochemical analysis unit 1. However, fluorescence data may be selectively recorded in a number of the absorptive regions 4 of the biochemical analysis unit 1 by selectively hybridizing a substance derived from a living organism and labeled with a hapten such as digoxigenin with specific labeling substances such as cDNAs fixed in a number of the absorptive regions 4 of the biochemical analysis unit 1 and further binding an antibody to the hapten labeled with an enzyme which generates a fluorescence substance when it contacts a fluorescent substrate with the hapten labeling a substance derived from a living organism and selectively hybridized with the specific binding substances fixed in number of the absorptive regions 4 of the biochemical analysis unit 1 by an antigen-antibody reaction.

Further, in the above described embodiments, although the hybridization reaction solution contains a substance derived from a living organism and labeled with a radioactive labeling substance, a substance derived from a living organism and labeled with a fluorescent substance and a substance derived from a living organism and labeled with a hapten such as digoxigenin, it is not absolutely necessary for the hybridization reaction solution to contain the hybridization reaction solution contains a substance derived from a living organism and labeled with a radioactive labeling substance, a substance derived from a living organism and labeled with a fluorescent substance and a substance derived from a living organism and labeled with a hapten such as digoxigenin and it is sufficient for the hybridization reaction solution to contain a substance derived from a living organism and labeled with at least one of a radioactive labeling substance, a fluorescent substance and a hapten such as digoxigenin.

Moreover, in the above described embodiments, as specific binding substances, cDNAs each of which has a known base sequence and is different from the others are used. However, specific binding substances usable in the present invention are not limited to cDNAs but all specific binding substances capable of specifically binding with a substance derived from a living organism such as a cell, virus, hormone, tumor marker, enzyme, antibody, antigen, abzyme, other protein, a nuclear acid, cDNA, DNA, RNA or the like and whose sequence, base length, composition and the like are known, can be employed in the present invention as a specific binding substance.

Further, in the embodiment shown in Figure 3, the hybridization reaction solution, the cleaning solution or the antibody solution containing an antibody to the hapten such as digoxigenin labeled with an enzyme which generates chemiluminescence emission when it contacts a chemiluminescent substrate contained in the space on one side of the biochemical analysis unit 1 held at the biochemical analysis unit holding section 7 of the reaction vessel 8 is pressurized and forcibly moved using the piston 15 of the syringe 16 so as to cut through a number of the absorptive regions 4 formed in the substrate 2 of the biochemical analysis unit 1 and in the embodiment shown in Figure 18, the hybridization reaction solution, the cleaning solution or the antibody solution containing an antibody to the hapten such as digoxigenin labeled with an enzyme which generates chemiluminescence emission when it contacts a chemiluminescent substrate contained in the space on one side of the biochemical analysis unit 1 held at the biochemical analysis unit holding section 130 of the reaction vessel 131 is pressurized and forcibly moved by alternately deforming the diaphragm 135 and the diaphragm 136 between the position indicated by the solid line and the position indicated by the broken line in Figure 18 so as to cut through a number of the absorptive regions 4 formed in the substrate 2 of the biochemical analysis unit 1. However, the hybridization reaction solution, the cleaning solution or the antibody solution containing an antibody to the hapten such as digoxigenin labeled with an enzyme which generates chemiluminescence emission when it contacts a chemiluminescent substrate contained in the space on one side of the biochemical analysis unit 1 held at the biochemical analysis unit holding section 7, 130 of the reaction vessel 8, 131 can be pressurized and forcibly moved by any of various methods so as to cut through a number of the absorptive regions 4 formed in the substrate 2 of the biochemical analysis unit 1 and it is not absolutely necessary to pressurize and forcibly move the hybridization reaction solution, the cleaning solution or the antibody solution containing an antibody to the hapten such as digoxigenin labeled with an enzyme which generates chemiluminescence emission when it contacts a chemiluminescent substrate contained in the space on one side of the biochemical analysis unit 1 held at the biochemical analysis unit holding section 7, 130 of the reaction vessel 8, 131 by using the piston 15 of the syringe 16 or alternately deforming the diaphragm 135 and the diaphragm 136 between the position indicated by a solid line and the position indicated by the broken line in Figure 18 so as to cut through a number of the absorptive regions 4 formed in the substrate 2 of the biochemical analysis unit 1.

Furthermore, in the above described embodiments, although the reactor is constituted so as to forcibly move in the vertical direction the hybridization reaction solution, the cleaning solution or the antibody solution containing an antibody to the hapten such as digoxigenin labeled with an enzyme which generates chemiluminescence emission when it contacts a chemiluminescent substrate, it is not absolutely necessary to constitute the reactor so as to forcibly move in the vertical direction the hybridization reaction solution, the cleaning solution or the antibody solution containing an antibody to the hapten such as digoxigenin labeled with an enzyme which generates chemiluminescence emission when it contacts a chemiluminescent substrate and the direction for moving the hybridization reaction solution, the cleaning solution and the antibody solution containing an antibody to the hapten such as digoxigenin labeled with an enzyme which generates chemiluminescence emission when it contacts a chemiluminescent substrate can be arbitrarily selected insofar as the reactors are constituted so as to be able to forcibly move the hybridization reaction solution, the cleaning solution and the antibody solution containing an antibody to the hapten such as digoxigenin labeled with an enzyme which generates chemiluminescence emission when it contacts a chemiluminescent substrate from one side of the biochemical analysis unit 1 held by the biochemical analysis unit holding section 7, 130 toward the other side thereof and forcibly move the hybridization reaction solution, the cleaning solution and the antibody solution containing an antibody to the hapten such as digoxigenin labeled with an enzyme which generates chemiluminescence emission when it contacts a chemiluminescent substrate from the other side of the biochemical analysis unit 1 held by the biochemical analysis unit holding section 7, 130 toward the one side thereof.

Moreover, in the embodiment shown in Figure 18, although each of the diaphragm 135 formed in the upper half portion 133 and the diaphragm 136 formed in the lower half portion 134 is formed of a metal plate having flexibility, it is not absolutely necessary to form each of diaphragms 135, 136 of a metal plate having flexibility and the diaphragms 135, 136 may be formed of a plastic material such as polypropylene insofar as it has flexibility.

Furthermore, in the embodiment shown in Figure 18, although the diaphragm 135 and the diaphragm 136 are deformed using the air cylinder, it is not absolutely necessary to deform the diaphragm 135 and the diaphragm 136 using an air cylinder and the diaphragm 135 and the diaphragm 136 can be deformed by any of various means.

Further, in the above described embodiments, although 19,200 substantially circular absorptive regions 4 having a size of about 0.01 mm² are formed in the substrate 2 of the biochemical analysis unit 1 in a regular pattern and in the manner of a matrix, the shape of each of the absorptive regions 4 is not limited to substantially a circular shape but may be formed in an arbitrary shape, for example, a rectangular shape.

Moreover, in the above described embodiments, although 19,200 substantially circular absorptive regions 4 having a size of about 0.01 mm² are formed in the substrate 2 of the biochemical analysis unit 1 in a regular pattern and in the manner of matrix, the number or size of the absorptive regions 4 may be arbitrarily selected in accordance with the purpose. Preferably, 10 or more of the absorptive regions 4 having a size of 5 mm² or less are formed in the biochemical analysis unit 1 at a density of 10/ cm² or greater.

Further, in the above described embodiments, although 19,200 substantially circular absorptive regions 4 having a size of about 0.01 mm² are formed in the substrate 2 of the biochemical analysis unit 1 in a regular pattern and in the manner of matrix, it is not absolutely necessary to form the absorptive regions 4 in the biochemical analysis unit 1 in a regular pattern.

Furthermore, in the above described embodiments, although the biochemical analysis unit 1 includes a number of the absorptive regions 4 formed by charging nylon-6,6 in a number of the through-hole 3 formed in the substrate 2 made of stainless steel, it is not absolutely necessary to form a number of the absorptive regions 4 of the biochemical analysis unit 1 of nylon-6, 6 but a number of the absorptive regions 4 of the biochemical analysis unit 1 may be formed instead of nylon-6, 6 of a porous carbon material such as an activated carbon or materials including nylons such as nylon-6, nylon-4,10; cellulose derivatives such as nitrocellulose, acetyl cellulose and butyric-acetyl cellulose; collagen; alginic acids such as alginic acid, calcium alginate and alginic acid/poly-L-lysine polyionic complex; polyolefins such as polyethylene, polypropylene; polyvinyl chloride; polyvinylidene chloride; polyfluorides such as polyvinylidene fluoride and polytetrafluoride; and copolymers or composite materials thereof. A number of the absorptive regions 4 of the biochemical analysis unit 1 may be formed of inorganic porous materials including metals such as platinum, gold, iron, silver, nickel, aluminum and the like; metal oxides such as alumina, silica, titania, zeolite and the like; metal salts such as hydroxy apatite, calcium sulfate and the like; and composite materials thereof, or a plurality of fiber bundles.

Moreover, in the above described embodiments, although the biochemical analysis unit 1 includes the substrate made of stainless steel, it is not absolutely necessary to make the substrate 2 of the biochemical analysis unit 1 of stainless steel but the substrate 2 of the biochemical analysis unit 1 may be made of other kinds of material. The substrate 2 of the biochemical analysis unit 1 is preferably made of material capable of attenuating light energy and radiation energy but the material for forming the substrate 2 of the biochemical analysis unit 1 is not particularly limited. The substrate 2 of the biochemical analysis unit 1 can be formed of either inorganic compound material or organic compound material and is preferably formed of a metal material, a ceramic material or a plastic material. Illustrative examples of inorganic compound materials preferably usable for forming the substrate 2 of the biochemical analysis unit 1 and having a property of attenuating light energy and radiation energy include metals such as gold, silver, copper, zinc, aluminum, titanium, tantalum, chromium, steel, nickel, cobalt, lead, tin, selenium and the like; alloys such as brass, stainless, bronze and the like; silicon materials such as silicon, amorphous silicon, glass, quartz, silicon carbide, silicon nitride and the like; metal oxides such as aluminum oxide, magnesium oxide, zirconium oxide and the like; and inorganic salts such as tungsten carbide, calcium carbide, calcium sulfate, hydroxy apatite, gallium arsenide and the like. These may have either a monocrystal structure or a polycrystal sintered structure such as amorphous, ceramic or the like. High molecular compounds are preferably used as organic compound material for forming the substrate 2 of the biochemical analysis unit 1 and having a property of attenuating light energy and radiation energy and illustrative examples thereof include polyolefins such as polyethylene, polypropylene and the like; acrylic resins such as polymethyl methacrylate, polybutylacrylate/polymethyl methacrylate copolymer and the like; polyacrylonitrile; polyvinyl chloride; polyvinylidene chloride; polyvinylidene fluoride; polytetrafluoroethylene; polychlorotrifluoroethylene; polycarbonate; polyesters such as polyethylene naphthalate, polyethylene terephthalate and the like; nylons such as nylon-6, nylon-6,6, nylon-4, 10 and the like; polyimide; polysulfone; polyphenylene sulfide; silicon resins such as polydiphenyl siloxane and the like; phenol resins such as novolac and the like; epoxy resin; polyurethane; polystyrene, butadiene-styrene copolymer; polysaccharides such as cellulose, acetyl cellulose, nitrocellulose, starch, calcium alginate, hydroxypropyl methyl cellulose and the like; chitin; chitosan; urushi (Japanese lacquer); polyamides such as gelatin, collagen, keratin and the like; and copolymers of these high molecular materials. These may be a composite compound, and metal oxide particles, glass fiber or the like may be added thereto as occasion demands. Further, an organic compound material may be blended therewith.

Further, in the above described embodiments, although a number of the absorptive regions 4 of the biochemical analysis unit 1 are formed by charging nylon-6, 6 in a number of the through-hole 3 formed in the substrate 2, a number of the absorptive regions 4 may be formed by pressing an absorptive membrane formed of an absorptive material such as nylon-6, 6 into a number of through-holes 3 formed in the substrate 2.

Furthermore, in the above described embodiments, although a number of the absorptive regions 4 of the biochemical analysis unit 1 are formed by charging nylon-6, 6 in a number of the through-hole 3 formed in the substrate 2, it is possible to bring a substrate formed with a number of through-holes into close contact with at least one surface of an absorptive substrate formed of an absorptive material such as a membrane filter and to form a number of absorptive regions by the absorptive substrate within a number of the through-holes formed in the substrate.

Further, in the above described embodiments, although a number of the absorptive regions 4 of the biochemical analysis unit 1 are formed by charging nylon-6, 6 in a number of the through-hole 3 formed in the substrate 2, it is possible it is possible instead to form a number of absorptive regions so as to be spaced from each other by dropping a solution containing specific binding substances in a regular pattern onto different regions of an absorptive substrate formed of an absorptive material such as a membrane filter.

According to the present invention, it is possible to provide a method for conducting a receptor-ligand association reaction which can efficiently associate a ligand or receptor with receptors or ligands fixed in spot-like regions of a biochemical analysis unit and produce biochemical analysis data having an excellent quantitative characteristic with good repeatability.

## Claims

1. A method for conducting a receptor-ligand association reaction comprising steps of pressurizing a reaction solution containing a ligand or receptor labeled with a labeling substance and forcibly feeding the reaction solution so as to cut through a plurality of absorptive regions formed in a biochemical analysis unit so as to be spaced from each other and containing receptors or ligands, thereby selectively associating the ligand or receptor contained in the reaction solution with the receptors or ligands contained in the plurality of absorptive regions of the biochemical analysis unit, wherein the biochemical analysis unit includes a substrate formed with a plurality of through-holes spaced apart from each other and the plurality of absorptive regions are formed by charging an absorptive material in the plurality of through-holes formed in the substrate so that a whole volume or most volume defined by each of the through-holes is filled with the absorptive material and causing the absorptive material charged in the plurality of through-holes to contain receptors or ligands.

2. A method for conducting a receptor-ligand association reaction in accordance with Claim 1 which comprises a step of pressurizing the reaction solution using a syringe, thereby forcibly feeding the reaction solution so as to cut through the plurality of absorptive regions formed in the biochemical analysis unit.

3. A method for conducting a receptor-ligand association reaction in accordance with Claim 1 which comprises a step of deforming diaphragms facing each other to pressurize the reaction solution, thereby forcibly feeding the reaction solution so as to cut through the plurality of absorptive regions formed in the biochemical analysis unit.

4. A method for conducting a receptor-ligand association reaction in accordance with any one of Claims 1 to 3 wherein the reaction solution contains a ligand or receptor labeled with at least one kind of labeling substance selected from a group consisting of a radioactive labeling substance, a fluorescent substance and a labeling substance which generates chemiluminescence emission when it contacts a chemiluminescent substrate.

5. A method for conducting a receptor-ligand association reaction in accordance with any one of Claims 1 to 4 which comprises steps of pressurizing a reaction solution containing a substance derived from a living organism and labeled with a labeling substance and forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with a labeling substance with the specific binding substances contained in the plurality of absorptive regions of the biochemical analysis unit.

6. A method for conducting a receptor-ligand association reaction in accordance with any one of Claims 1 to 4 which comprises steps of pressurizing a reaction solution containing an antibody or an antigen labeled with a labeling substance and forcibly feeding the reaction solution so as to cut through the plurality of absorptive regions formed in the biochemical analysis unit so as to be spaced apart from each other and containing an antigen or an antibody, thereby binding the antibody or the antigen labeled with a labeling substance with the antigen or the antibody contained in the plurality of absorptive regions of the biochemical analysis unit.

7. A method for conducting a receptor-ligand association reaction in accordance with any one of Claims 1 to 4 which comprises steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, pressurizing an antibody solution containing an antibody to the hapten labeled with a labeling substance and forcibly feeding the antibody solution so as to cut through the plurality of absorptive regions of the biochemical analysis unit, thereby binding the antibody labeled with the labeling substance with the hapten contained in the plurality of absorptive regions of the biochemical analysis unit by an antigen-antibody reaction.

8. A method for conducting a receptor-ligand association reaction in accordance with Claim 7 which comprises steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, and pressurizing an antibody solution containing an antibody to the hapten labeled with an enzyme which generates chemiluminescence emission when it contacts a chemiluminescent substrate so as to cut through the plurality of absorptive regions formed in the biochemical analysis unit, thereby binding the antibody labeled with the enzyme with the hapten contained in the plurality of absorptive regions of the biochemical analysis unit by an antigen-antibody reaction.

9. A method for conducting a receptor-ligand association reaction in accordance with Claim 7 which comprises steps of pressurizing a reaction solution containing a substance derived from a living organism and labeled with a hapten, forcibly feeding the reaction solution so as to cut through the plurality of absorptive regions formed in the biochemical analysis unit so as to be spaced apart from each other and containing specific binding substances whose structure or characteristics are known, thereby selectively hybridizing the substance derived from a living organism and labeled with the hapten with the specific binding substances contained in the plurality of absorptive regions of the biochemical analysis unit, and pressurizing an antibody solution containing an antibody to the hapten labeled with an enzyme which generates a fluorescent substance when it contacts a fluorescent substrate so as to cut through the plurality of absorptive regions formed in the biochemical analysis unit, thereby binding the antibody labeled with the enzyme with the hapten contained in the plurality of absorptive regions of the biochemical analysis unit by an antigen-antibody reaction.

10. A method for conducting a receptor-ligand association reaction in accordance with any one of claims 1 to 9, wherein the plurality of absorptive regions are formed by pressing an absorptive membrane containing an absorptive material into the plurality of through-holes formed in the substrate and causing the absorptive material pressed into the plurality of through-holes to contain receptors or ligands.

11. A method for conducting a receptor-ligand association reaction in accordance with any one of claims 1 to 10, wherein the substrate of the biochemical analysis unit has a property of reducing the energy of radiation to 1/5 or less when the radiation travels in the substrate by a distance equal to that between neighboring absorptive regions.

12. A method for conducting a receptor-ligand association reaction in accordance with any one of claims 1 to 11, wherein the substrate of the biochemical analysis unit has a property of reducing the energy of light to 1/5 or less when the light travels in the substrate by a distance equal to that between neighboring absorptive regions.

13. A method for conducting a receptor-ligand association reaction in accordance with any one of Claims 1 to 12 wherein the substrate of the biochemical analysis unit is made of a metal material, a ceramic material or a plastic material.

14. A method for conducting a receptor-ligand association reaction in accordance with any one of Claims 1 to 13 wherein the biochemical analysis unit is formed with 10 or more absorptive regions.

15. A method for conducting a receptor-ligand association reaction in accordance with any one of Claims 1 to 14 wherein each of the plurality of absorptive regions of the biochemical analysis unit has a size of less than 5 mm²

16. A method for conducting a receptor-ligand association reaction in accordance with any one of Claims 1 to 15 wherein the plurality of absorptive regions are formed in the biochemical analysis unit at a density of 10 or more per cm².

## Patentansprüche

1. Verfahren zur Durchführung einer Rezeptor-Ligand-Assoziationsreaktion, aufweisend die Schritte des Druckbeaufschlagens einer Reaktionslösung, die einen Liganden oder Rezeptor, der mit einer Markierungssubstanz markiert ist, enthält, und des kraftvoll Zuführens der Reaktionslösung, so dass sie eine Mehrzahl absorptionsfähiger Bereiche, die in einer biochemischen Analyseeinheit so ausgebildet sind, dass sie voneinander beabstandet sind und Rezeptoren oder Liganden enthalten, durchdringt, wodurch der in der Reaktionslösung enthaltene Ligand oder Rezeptor selektiv an die in der Mehrzahl absorptionsfähiger Bereiche der biochemischen Analyseeinheit enthaltenen Rezeptoren oder Liganden assoziiert, wobei die biochemische Analyseeinheit ein Substrat aufweist, das mit einer Mehrzahl von durchgehenden Öffnungen, die voneinander beabstandet sind, ausgebildet ist, und wobei die Mehrzahl der absorptionsfähigen Bereiche ausgebildet ist durch Einbringen eines absorptionsfähigen Materials in die Mehrzahl von in dem Substrat ausgebildeten durchgehenden Öffnungen, so dass das gesamte Volumen oder das meiste Volumen, das von jeder der durchgehenden Öffnungen definiert wird, mit dem absorptionsfähigen Material gefüllt ist, und durch Veranlassen des in die Mehrzahl von durchgehenden Öffnungen eingebrachten absorptionsfähigen Materials, Rezeptoren oder Liganden zu enthalten.

2. Verfahren zur Durchführung einer Rezeptor-Ligand-Assoziationsreaktion gemäß Anspruch 1, das einen Schritt des Druckbeaufschlagens der Reaktionslösung unter Verwendung einer Spritze aufweist, wodurch die Reaktionslösung kraftvoll zugeführt wird, so dass sie die Mehrzahl von absorptionsfähigen Bereichen, die in der biochemischen Analyseeinheit ausgebildet sind, durchdringt.

3. Verfahren zur Durchführung einer Rezeptor-Ligand-Assoziationsreaktion gemäß Anspruch 1, das einen Schritt des Verformens von einander zugewandten Diaphragmen, um die Reaktionslösung mit Druck zu beaufschlagen, aufweist, wodurch die Reaktionslösung kraftvoll zugeführt wird, so dass sie die Mehrzahl von absorptionsfähigen Bereichen, die in der biochemischen Analyseeinheit ausgebildet sind, durchdringt.

4. Verfahren zur Durchführung einer Rezeptor-Ligand-Assoziationsreaktion gemäß einem der Ansprüche 1 bis 3, bei dem die Reaktionslösung einen Liganden oder Rezeptor enthält, der mit mindestens einer Art von Markierungssubstanz markiert ist, die ausgewählt ist aus der Gruppe, die aus einer radioaktiven Markierungssubstanz, einer fluoreszierenden Substanz und einer Markierungssubstanz, die eine Chemilumineszenzemission erzeugt, wenn sie mit einem chemilumineszierenden Substrat in Kontakt kommt, besteht.

5. Verfahren zur Durchführung einer Rezeptor-Ligand-Assoziationsreaktion gemäß einem der Ansprüche 1 bis 4, das die Schritte aufweist des Druckbeaufschlagens einer Reaktionslösung, die eine von einem lebenden Organismus stammende und mit einer Markierungssubstanz markierte Substanz enthält, und des kraftvoll Zuführens der Reaktionslösung, so dass sie die Mehrzahl von absorptionsfähigen Bereichen, die in der biochemischen Analyseeinheit so ausgebildet sind, dass sie voneinander beabstandet sind, und die spezifische Bindungssubstanzen, deren Struktur oder Eigenschaften bekannt sind, enthalten, durchdringt, wodurch die von einem lebenden Organismus stammende und mit einer Markierungssubstanz markierte Substanz selektiv an die spezifischen Bindungssubstanzen, die in der Mehrzahl von absorptionsfähigen Bereichen der biochemischen Analyseeinheit enthalten sind, hybridisiert.

6. Verfahren zur Durchführung einer Rezeptor-Ligand-Assoziationsreaktion gemäß einem der Ansprüche 1 bis 4, das die Schritte aufweist des Druckbeaufschlagens einer Reaktionslösung, die einen Antikörper oder ein Antigen, der (das) mit einer Markierungssubstanz markiert ist, enthält, und des kraftvoll Zuführens der Reaktionslösung, so dass sie die Mehrzahl von absorptionsfähigen Bereichen, die in der biochemischen Analyseeinheit so ausgebildet sind, dass sie voneinander beabstandet sind, und die ein Antigen oder einen Antikörper enthalten, durchdringt, wodurch der Antikörper oder das Antigen, der (das) mit einer Markierungssubstanz markiert ist, an das Antigen oder den Antikörper, das (der) in der Mehrzahl von absorptionsfähigen Bereichen der biochemischen Analyseeinheit enthalten ist, gebunden wird.

7. Verfahren zur Durchführung einer Rezeptor-Ligand-Assoziationsreaktion gemäß einem der Ansprüche 1 bis 4, das die Schritte aufweist des Druckbeaufschlagens einer Reaktionslösung, die eine Substanz, die von einem lebenden Organismus stammt und mit einem Hapten markiert ist, enthält, des kraftvoll Zuführens der Reaktionslösung, so dass sie die Mehrzahl von absorptionsfähigen Bereichen, die in der biochemischen Analyseeinheit so ausgebildet sind, dass sie voneinander beabstandet sind, und die spezifische Bindungssubstanzen, deren Struktur oder Eigenschaften bekannt sind, enthalten, durchdringt, wodurch die von einem lebenden Organismus stammende und mit dem Hapten markierte Substanz selektiv an die spezifischen Bindungssubstanzen, die in der Mehrzahl von absorptionsfähigen Bereichen der biochemischen Analyseeinheit enthalten sind, hybridisiert, des Druckbeaufschlagens einer Antikörper-Lösung, die einen mit einer Markierungssubstanz markierten Antikörper gegen das Hapten enthält, und des kraftvoll Zuführens der Antikörper-Lösung, so dass sie die Mehrzahl von absorptionsfähigen Bereichen der biochemischen Analyseeinheit durchdringt, wodurch der mit der Markierungssubstanz markierte Antikörper durch eine Antigen-Antikörper-Reaktion an das in der Mehrzahl von absorptionsfähigen Bereichen der biochemischen Analyseeinheit enthaltene Hapten bindet.

8. Verfahren zur Durchführung einer Rezeptor-Ligand-Assoziationsreaktion gemäß Anspruch 7, das die Schritte aufweist des Druckbeaufschlagens einer Reaktionslösung, die eine von einem lebenden Organismus stammende und mit einem Hapten markierte Substanz enthält, des kraftvoll Zuführens der Reaktionslösung, so dass sie die Mehrzahl der absorptionsfähigen Bereiche, die so in der biochemischen Analyseeinheit ausgebildet sind, dass sie voneinander beabstandet sind, und die spezifische Bindungssubstanzen, deren Struktur oder Eigenschaften bekannt sind, enthalten, durchdringt, wodurch die von einem lebenden Organismus stammende und mit dem Hapten markierte Substanz selektiv an die in der Mehrzahl von absorptionsfähigen Bereichen der biochemischen Analyseeinheit enthaltenen spezifischen Bindungssubstanzen hybridisiert, und des Druckbeaufschlagens einer Antikörper-Lösung, die einen mit einem Enzym, das eine Chemilumineszenzemission erzeugt, wenn es mit einem chemilumineszierenden Substrat in Kontakt kommt, markierten Antikörper gegen das Hapten enthält, so dass sie die Mehrzahl von in der biochemischen Analyseeinheit ausgebildeten absorptionsfähigen Bereichen durchdringt, wodurch der mit dem Enzym markierte Antikörper durch eine Antigen-Antikörper-Reaktion an das in der Mehrzahl von absorptionsfähigen Bereichen der biochemischen Analyseeinheit enthaltene Hapten gebunden wird.

9. Verfahren zur Durchführung einer Rezeptor-Ligand-Assoziationsreaktion gemäß Anspruch 7, das die Schritte aufweist des Druckbeaufschlagens einer Reaktionslösung, die eine von einem lebenden Organismus stammende und mit einem Hapten markierte Substanz enthält, des kraftvoll Zuführens der Reaktionslösung, so dass sie die Mehrzahl von absorptionsfähigen Bereichen, die in der biochemischen Analyseeinheit so ausgebildet sind, dass sie voneinander beabstandet sind, und die spezifische Bindungssubstanzen, deren Struktur oder Eigenschaften bekannt sind, enthalten, durchdringt, wodurch die von einem lebenden Organismus stammende und mit dem Hapten markierte Substanz selektiv an die in der Mehrzahl von absorptionsfähigen Bereichen der biochemischen Analyseeinheit enthaltenen spezifischen Bindungssubstanzen hybridisiert wird, und des Druckbeaufschlagens einer Antikörper-Lösung, die einen mit einem Enzym, das eine fluoreszierende Substanz erzeugt, wenn es mit einem fluoreszierenden Substrat in Kontakt kommt, markierten Antikörper gegen das Hapten enthält, so dass sie die Mehrzahl von in der biochemischen Analyseeinheit ausgebildeten absorptionsfähigen Bereichen durchdringt, wodurch der mit dem Enzym markierte Antikörper durch eine Antigen-Antikörper-Reaktion an das in der Mehrzahl von absorptionsfähigen Bereichen der biochemischen Analyseeinheit enthaltene Hapten gebunden wird.

10. Verfahren zur Durchführung einer Rezeptor-Ligand-Assoziationsreaktion gemäß einem der Ansprüche 1 bis 9, bei dem die Mehrzahl von absorptionsfähigen Bereichen gebildet wird durch Pressen einer absorptionsfähigen Membran, die ein absorptionsfähiges Material enthält, in die Mehrzahl von in dem Substrat ausgebildeten durchgehenden Öffnungen, und durch Veranlassen des in die Mehrzahl von durchgehenden Öffnungen gepreßten absorptionsfähigen Materials, Rezeptoren oder Liganden zu enthalten.

11. Verfahren zur Durchführung einer Rezeptor-Ligand-Assoziationsreaktion gemäß einem der Ansprüche 1 bis 10, bei dem das Substrat der biochemischen Analyseeinheit die Eigenschaft hat, die Energie von Strahlung auf 1/5 oder weniger zu verringern, wenn die Strahlung in dem Substrat eine Strecke durchläuft, die gleich derjenigen zwischen benachbarten absorptionsfähigen Bereichen ist.

12. Verfahren zur Durchführung einer Rezeptor-Ligand-Assoziationsreaktion gemäß einem der Ansprüche 1 bis 11, bei dem das Substrat der biochemischen Analyseeinheit die Eigenschaft hat, die Energie von Licht auf 1/5 oder weniger zu verringern, wenn das Licht in dem Substrat eine Strecke durchläuft, die gleich derjenigen zwischen benachbarten absorptionsfähigen Bereichen ist.

13. Verfahren zur Durchführung einer Rezeptor-Ligand-Assoziationsreaktion gemäß einem der Ansprüche 1 bis 12, bei dem das Substrat der biochemischen Analyseeinheit aus einem Metallmaterial, einem Keramikmaterial oder einem Kunststoffmaterial hergestellt ist.

14. Verfahren zur Durchführung einer Rezeptor-Ligand-Assoziationsreaktion gemäß einem der Ansprüche 1 bis 13, bei dem die biochemische Analyseeinheit mit 10 oder mehr absorptionsfähigen Bereichen ausgebildet ist.

15. Verfahren zur Durchführung einer Rezeptor-Ligand-Assoziationsreaktion gemäß einem der Ansprüche 1 bis 14, bei dem jeder Bereich der Mehrzahl von absorptionsfähigen Bereichen der biochemischen Analyseeinheit eine Größe von weniger als 5 mm² hat.

16. Verfahren zur Durchführung einer Rezeptor-Ligand-Assoziationsreaktion gemäß einem der Ansprüche 1 bis 15, bei dem die Mehrzahl von absorptionsfähigen Bereichen in der biochemischen Analyseeinheit mit einer Dichte von 10 oder mehr pro cm² ausgebildet sind.

## Revendications

1. Procédé pour effectuer une réaction de liaison entre récepteurs et ligands comprenant des étapes de pressurisation d'une solution réactionnelle contenant un ligand ou un récepteur marqué avec une substance de marquage et d'introduction forcée de la solution réactionnelle de manière à ce qu'elle traverse une pluralité de régions absorbantes formées dans une unité d'analyses biochimiques de manière à ce qu'elles soient espacées les unes des autres et contenant des récepteurs ou des ligands, associant ainsi de manière sélective le ligand ou le récepteur contenu dans la solution réactionnelle avec les récepteurs ou les ligands contenus dans la pluralité de régions absorbantes de l'unité d'analyses biochimiques, dans lequel l'unité d'analyses biochimiques comprend un substrat formé d'une pluralité de trous traversants séparés les uns des autres et la pluralité de régions absorbantes est formée en chargeant un matériau absorbant dans la pluralité de trous traversants formés dans le substrat de sorte qu'un volume total ou que la plupart d'un volume défini par chacun des trous traversants soit rempli du matériau absorbant et provoquant le fait que le matériau absorbant chargé dans la pluralité de trous traversants contienne des récepteurs ou des ligands.

2. Procédé pour effectuer une réaction de liaison entre récepteurs et ligands selon la revendication 1 qui comprend une étape de pressurisation de la solution réactionnelle en utilisant une seringue, pour introduire ainsi de manière forcée la solution réactionnelle de manière à ce qu'elle traverse la pluralité de régions absorbantes formées dans l'unité d'analyses biochimiques.

3. Procédé pour effectuer une réaction de liaison entre récepteurs et ligands selon la revendication 1 qui comprend une étape consistant à déformer les membranes se faisant face pour pressuriser la solution réactionnelle, et introduire ainsi de manière forcée la solution réactionnelle de manière à ce qu'elle traverse la pluralité des régions absorbantes formées dans l'unité d'analyses biochimiques.

4. Procédé pour effectuer une réaction de liaison entre récepteurs et ligands selon l'une quelconque des revendications 1 à 3 dans lequel la solution réactionnelle contient un ligand ou un récepteur marqué avec au moins un type d'une substance de marquage choisi parmi un groupe constitué d'une substance de marquage radioactive, d'une substance fluorescente et d'une substance de marquage qui génère une émission de chimioluminescence quand elle entre en contact avec un substrat chimioluminescent.

5. Procédé pour effectuer une réaction de liaison entre récepteurs et ligands selon l'une quelconque des revendications 1 à 4 qui comprend des étapes de pressurisation d'une solution réactionnelle contenant une substance dérivée d'un organisme vivant et marquée avec une substance de marquage et d'introduction forcée de la solution réactionnelle de manière à ce qu'elle traverse la pluralité de régions absorbantes formées dans l'unité d'analyses biochimiques de manière à ce qu'elles soient espacées les unes des autres et contenant des substances de liaisons spécifiques dont la structure ou les caractéristiques sont connues, hybridant ainsi sélectivement la substance dérivée d'un organisme vivant et marquée avec une substance de marquage aux substances de liaisons spécifiques contenues dans la pluralité de régions absorbantes de l'unité d'analyses biochimiques.

6. Procédé pour effectuer une réaction de liaison entre récepteurs et ligands selon l'une quelconque des revendications 1 à 4 qui comprend des étapes de pressurisation d'une solution réactionnelle contenant un anticorps ou un antigène marqué avec une substance de marquage et d'introduction forcée de la solution réactionnelle de manière à ce qu'elle traverse la pluralité de régions absorbantes formées dans l'unité d'analyses biochimiques de manière à ce qu'elles soient espacées les unes des autres et contenant un antigène ou un anticorps, liant ainsi l'anticorps ou l'antigène marqué avec une substance de marquage avec l'antigène ou l'anticorps contenu dans ladite pluralité de régions absorbantes de l'unité d'analyses biochimiques.

7. Procédé pour effectuer une réaction de liaison entre récepteurs et ligands selon l'une quelconque des revendications 1 à 4 qui comprend des étapes de pressurisation d'une solution réactionnelle contenant une substance dérivée d'un organisme vivant et marquée avec un haptène, d'introduction forcée de la solution réactionnelle de manière à ce qu'elle traverse la pluralité des régions absorbantes formées dans l'unité d'analyses biochimiques de manière à ce qu'elles soient séparées les unes des autres et contenant des substances de liaisons spécifiques dont la structure ou les caractéristiques sont connues, hybridant ainsi sélectivement la substance dérivée d'un organisme vivant et marquée avec l'haptène aux substances de liaisons spécifiques contenues dans la pluralité de régions absorbantes de l'unité d'analyses biochimiques, pressurisant une solution d'anticorps contenant un anticorps anti-haptène marqué avec une substance de marquage et introduisant de manière forcée la solution d'anticorps de manière à ce qu'elle traverse la pluralité de régions absorbantes de l'unité d'analyses biochimiques, liant ainsi l'anticorps marqué avec la substance de marquage à l'haptène contenu dans la pluralité de régions absorbantes de l'unité d'analyses biochimiques par une réaction antigène-anticoprs.

8. Procédé pour effectuer une réaction de liaison entre récepteurs et ligands selon la revendication 7 qui comprend des étapes de pressurisation d'une solution réactionnelle contenant une substance dérivée d'un organisme vivant et marquée avec un haptène, d'introduction forcée de la solution réactionnelle de manière à ce qu'elle traverse la pluralité de régions absorbantes formées dans l'unité d'analyses biochimiques de manière à ce qu'elles soient espacées les unes des autres et contenant des substances de liaisons spécifiques dont la structure ou les caractéristiques sont connues, hybridant ainsi sélectivement la substance dérivée d'un organisme vivant et marquée avec l'haptène aux substances de liaisons spécifiques contenues dans la pluralité des régions absorbantes de l'unité d'analyses biochimiques, et pressurisant une solution d'anticorps contenant un anticorps anti-haptène marqué avec une enzyme qui génère une émission de chimioluminescence quand il entre en contact avec un substrat chimioluminescent de manière à ce qu'elle traverse la pluralité des régions absorbantes formées dans l'unité d'analyses biochimiques, liant ainsi l'anticorps marqué avec l'enzyme à l'haptène contenu dans la pluralité des régions absorbantes de l'unité d'analyses biochimiques par une réaction antigène-anticoprs.

9. Procédé pour effectuer une réaction de liaison entre récepteurs et ligands selon la revendication 7 qui comprend des étapes de pressurisation d'une solution réactionnelle contenant une substance dérivée d'un organisme vivant et marquée avec l'haptène, d'introduction forcée de la solution réactionnelle de manière à ce qu'elle traverse la pluralité de régions absorbantes formées dans l'unité d'analyses biochimiques de manière à ce qu'elles soient espacées les unes des autres et contenant des substances de liaisons spécifiques dont la structure ou les caractéristiques sont connues, hybridant ainsi sélectivement la substance dérivée d'un organisme vivant et marquée avec l'haptène aux substances de liaisons spécifiques contenues dans la pluralité de régions absorbantes de l'unité d'analyses biochimiques, et pressurisant une solution d'anticorps contenant un anticorps anti-haptène marqué avec une enzyme qui génère une substance fluorescente quand elle entre en contact avec un substrat fluorescent de manière à ce qu'elle traverse la pluralité des régions absorbantes formées dans l'unité d'analyses biochimiques, liant ainsi l'anticorps marqué avec l'enzyme à l'haptène contenu dans la pluralité des régions absorbantes de l'unité d'analyses biochimiques par une réaction antigène-anticorps.

10. Procédé pour effectuer une réaction de liaison entre récepteurs et ligands selon l'une quelconque des revendications 1 à 9, dans lequel la pluralité des régions absorbantes sont formées en pressant une membrane absorbante contenant un matériau absorbant dans la pluralité de trous traversants formés dans le substrat et en provoquant le fait que le matériau absorbant pressé dans la pluralité de trous traversants contienne des récepteurs ou des ligands.

11. Procédé pour effectuer une réaction de liaison entre récepteurs et ligands selon l'une quelconque des revendications 1 à 10, dans lequel le substrat de l'unité d'analyses biochimiques a une propriété de réduction de l'énergie de rayonnement à 1/5 quand le rayonnement se déplace dans le substrat d'une distance égale à celle entre les régions absorbantes avoisinantes.

12. Procédé pour effectuer une réaction de liaison entre récepteurs et ligands selon l'une quelconque des revendications 1 à 11, dans lequel le substrat de l'unité d'analyses biochimiques a une propriété de réduction de l'énergie de la lumière à 1/5 quand la lumière se déplace dans le substrat d'une distance égale à celle entre les régions absorbantes avoisinantes.

13. Procédé pour effectuer une réaction de liaison entre récepteurs et ligands selon l'une quelconque des revendications 1 à 12 dans lequel le substrat de l'unité d'analyses biochimiques est constitué d'un matériau métallique, d'un matériau céramique ou d'un matériau plastique.

14. Procédé pour effectuer une réaction de liaison entre récepteurs et ligands selon l'une quelconque des revendications 1 à 13, dans lequel l'unité d'analyses biochimiques est formée avec 10 régions absorbantes ou plus.

15. Procédé pour effectuer- une réaction de liaison entre récepteurs et ligands selon l'une quelconque des revendications 1 à 14, dans lequel chacune de la pluralité des régions absorbantes de l'unité d'analyses biochimiques a une taille de moins de 5 mm².

16. Procédé pour effectuer une réaction de liaison entre récepteurs et ligands selon l'une quelconque des revendications 1 à 15, dans lequel la pluralité de régions absorbantes sont formées dans l'unité d'analyses biochimiques à une densité de 10 ou plus par cm².
